# EUROPEAN PATENT APPLICATION

(11) **EP 4 678 216 A1**
(43) Date of publication of application: **14.01.2026**
(21) Application number: 24770096.6
(22) Date of filing: 07.03.2024
(51) Int. Cl.: A61M 25/01, A61M 25/09, A61B 34/30

(54) **ELONGATED MEDICAL DEVICE MOVEMENT ASSEMBLY AND MEDICAL ROBOT USING SAME**

(30) Priority: 10.03.2023 KR 20230032057
(71) Applicant: XCATH, Inc., Houston, Texas 77054 (US)
(72) Inventor: JANG, Hyung Jun, Suwon-si, Gyeonggi-do 16420 (KR)
(74) Representative: Patentanwälte Ruff, Wilhelm, Beier, Dauster & Partner mbB
(86) International application number: PCT/IB2024/052190
(87) International publication number: WO 2024/189476

(57) **Abstract**

[Summary]

The present invention is to solve the problems of increased volume and manufacturing cost due to increased parts constituting the elongated medical device rotation assembly, as the elongated medical device rotation assembly exists outside the cassette, the problem of rotational force from the elongated medical device rotation assembly not being properly transmitted to the elongated medical device, the problem of twisting occurring when the elongated medical device rotates due to this, and the problem of the rotational direction of the elongated medical device rotation assembly being opposite to the direction in which the elongated medical device rotates inside the cassette.

An elongated medical device rotation assembly (400) according to one aspect of the present invention comprises a rotation member (401), a support member (500), and an offset member (660). The rotation member (401) rotates by driving an actuator. The support member (500) is coupled to the rotation member (401) and supports at least a portion of an elongated medical device (800) and rotates the elongated medical device (800) about its longitudinal axis in conjunction with the rotation of the rotation member (401). The support member (500) includes an offset member (660) for spacing at least a portion of the elongated medical device (800) apart from an imaginary line extending along the rotational axis of the rotation member (401) by a predetermined distance d on a surface on which the elongated medical device (800) is placed.

## Description

### [Technical Field]

The present invention relates to an elongated medical device movement assembly for moving forward, moving backward and rotating an elongated medical device, for example, a medical catheter or guidewire that can be inserted into a body, and a medical robot using the same, and more particularly, to an elongated medical device movement assembly for moving forward, moving backward and rotating an elongated medical device, thereby positioning a guidewire and catheter in a precise position within a body, e.g., a blood vessel, and enabling a practitioner to perform a desired procedure, and a medical robot using the same.

### [Background]

Catheters and guidewires can be used in various ways in medicine. The present invention can be used in minimally invasive vascular interventions. Minimally invasive vascular interventions are minimally invasive procedures aimed at treating vascular diseases or cancer, which are primarily performed by inserting a catheter through a blood vessel under X-ray fluoroscopy to reach the target organ or blood vessel for treatment. In order to move an elongated medical device, manipulations such as forward movement and rotation of the elongated medical device were required.

Blood vessels have a variety of shapes and contain foreign substances, such as edema. In addition, because blood flows through blood vessels, it was difficult to easily move elongated medical devices, such as catheters, to the target site. Furthermore, blood vessels are classified into various types, including arteries, veins and capillaries, and vary greatly in diameter, stiffness and degree of tortuosity. Moreover, if a wound occurs within a blood vessel during a procedure, it can lead to infection or bleeding. In light of the above, precise control of elongated medical devices has been a critical requirement in the industry. In particular, guidewires have been difficult to rotate due to their extremely thin size.

Accordingly, there is a need in the art for a technology that can rotate a guidewire as precisely as possible.

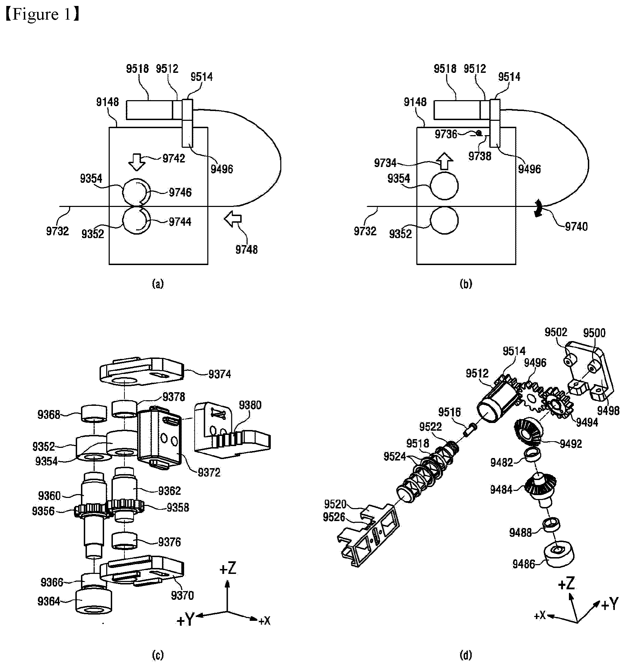

Figures 1a and 1b are schematic diagrams for explaining the forward movement and rotation of the conventional elongated medical devices, respectively.

A proximal cassette according to the prior art comprises a pinch and forward assembly and an elongated medical device rotation assembly. The pinch and forward assembly includes forward rollers (9352, 9354). The roller surfaces of the forward rollers (9352, 9354) face each other. During operation, the elongated medical device is positioned between the roller surfaces of the forward rollers (9352, 9354). Accordingly, the roller surfaces of the forward rollers (9352, 9354) come into contact with the elongated medical device and move the elongated medical device forward. Here, forward movement refers to supplying an elongated medical device to a human body or withdrawing an elongated medical device from a human body. The elongated medical device rotation assembly comprises a second spur gear (9496), a cap (9512), a plate spur gear (9514) and an elongated medical device connector (9518). Rotation of the second spur gear (9496) rotates the plate spur gear (9514) in the opposite direction, and thereby, the elongated medical device rotation assembly rotates the elongated medical device.

To move the guidewire (9732) forward, the pinch and forward assembly of the proximal cassette (9148) pinches the guidewire (9732). The forward roller (9354) is moved laterally so that the opposing forward roller (9352) applies an opposing force (e.g., pinch) to the guidewire (9732). The forward roller (9354) is moved toward the forward roller (9352) in the direction (9742) to the pinch position. With the guidewire (9732) pinched by the pinch and forward assembly, the pinch and forward assembly can move forward (e.g., feed into or retrieve from) the guidewire (9732). Rotations (9744, 9746) of the forward rollers (9352, 9354) can cause the guidewire (9732) to be fed into the body. Rotations of the forward rollers (9352, 9354) opposite to each of the illustrated rotations (9744, 9746) can cause the catheter to be withdrawn from the body.

Regardless of the movement (e.g., rotation, forward movement, and non-movement) of the elongated medical device (9732), the elongated medical device rotation assembly of the proximal cassette (9148) can remain mechanically coupled to the elongated medical device (9732). First, assuming that the proximal cassette (9148) is in a position where the elongated medical device (9732) is not moving, the pinch and forward assembly of the proximal cassette (9148) secures the elongated medical device (9732) between the forward rollers (9352, 9354) of the proximal cassette (9148). To rotate the elongated medical device (9732), the pinch and forward assembly releases the elongated medical device (9732). The forward roller (9354) of the proximal cassette (9148) is translated laterally in a direction (9734) such that the opposing forward roller (9352) does not exert an opposing force on the elongated medical device (9732). As the elongated medical device (9732) is released by the pinch and forward assembly of the proximal cassette (9148), the elongated medical device rotation assembly of the proximal cassette (9148) can rotate the elongated medical device (9732).

Figure 1c is an exploded perspective view of the pinch and forward assembly. Figure 1c is a drawing for explaining the operation of the pinch and forward assembly. Figure 1d is an exploded perspective view of the elongated medical device rotation assembly. Figure 1d is a drawing for explaining the operation of the elongated medical device rotation assembly.

The elongated medical device rotation assembly further comprises a drive bevel gear (9492) that is engaged with the drive bevel gear (9482), the first and second spur gears (9494, 9496), and the bracket (9498). The first spur gear (9494) meshes (engages) with the second spur gear (9496). The bevel drive gear (9492) is mechanically attached to the first spur gear (9494). The respective rotational axes of the bevel drive gear (9492) and the first spur gear (9494) are colinear. The elongated medical device rotation assembly comprises a cap (9512), a cap spur gear (9514), a collet (9516), an elongated medical device connector (9518), and a clamping bracket (9520). In the illustrated example, the plate spur gear (9514) is coupled to the cap (9512). The cap (9512) includes a threaded female connector. The elongated medical connector (9518) includes a threaded male connector (9522). When assembled, the threaded female connector of the cap (9512) is engaged with the threaded male connector (9522) of the elongated medical device connector (9518). When assembled, the collet (9516) is inserted into the tapered recess of the elongated medical device connector (9518), and the threaded female connector of the cap (9512) engages with the threaded male connector (9522) of the elongated medical device connector (9518). The clamping bracket (9520) holds the elongated medical device connector (9518) while the elongated medical device connector (9518) rotates. The rotation of the drive bevel gear (9492) rotates the first spur gear (9494) in the same direction. The rotation of the first spur gear (9494) rotates the second spur gear (9496) in the opposite direction, i.e., in the opposite direction. The rotation of the second spur gear (9496) rotates the plate spur gear (9514) in the opposite direction, which causes the elongated medical device to rotate when the collet (9516) is clamped on the elongated medical device extending therethrough.

As described above, the conventional elongated medical device pinch and forward assembly had to comprise forward spur gears (9356, 9358) to move the elongated medical device forward. In addition, the forward shaft (9360) had to be coupled to the forward spur gear (9356), and the forward shaft (9362) had to be coupled to the forward spur gear (9358). In addition, the forward spur gear (9356) and the forward spur gear (9358) had to be arranged on the forward shaft (9360) and the forward shaft (9362), respectively, and had to be structured to surround the forward shaft (9360) and the forward shaft (9362). In addition, the rotation of the forward shaft (9360) had to cause the rotation of the forward spur gear (9356), which had to cause the rotation of the forward spur gear (9358) and the forward shaft (9362) in the opposite rotational direction. Accordingly, the reverse rotation of the forward shaft (9360, 9362) caused the reverse rotation of the forward roller (9352, 9354), and at this pinch position, the forward roller (9352, 9354) had to be structured so that the elongated medical device would forward through the rotation of the forward roller (9352, 9354) as the forward roller pinched the elongated medical device.

Additionally, the elongated medical device rotation assembly had to comprise a cap (9512), a collet (9516), an elongated medical device connector (9518), and a clamping bracket (9520). The plate spur gear (9514) had to be coupled to the cap (9512), and the cap (9512) had to include a threaded female connector. The elongated medical device connector (9518) had to include a threaded male connector (9522). Additionally, during assembly, the threaded female connector of the cap (9512) had to engage with the threaded male connector (9522) of the elongated medical device connector (9518). Additionally, when assembled, the collet (9516) should have been inserted into the tapered recess of the elongated medical device connector (9518), and the threaded female connector of the cap (9512) should have been engaged with the threaded male connector (9522) of the elongated medical device connector (9518). The clamping bracket (9520) should have held the elongated medical device connector (9518) while the elongated medical device connector (9518) rotated.

Due to these circumstances, the prior art may cause the force with which the rollers pinch the elongated medical device to increase, thereby causing physical changes in the elongated medical device. For example, the elongated medical device may become thinner due to the pinching force of the rollers. Furthermore, in such cases, as the conventional art, if the forward spur gear is engaged to rotate the forward rollers and thereby forward the elongated medical device, the elongated medical device may not contact some of the rollers. In this case, the rollers will continue to spin unattended, and the elongated medical device may not move forward properly.

Furthermore, in the prior art, the rotation assembly of the elongated medical device was located externally. Consequently, separate space and components were required to enclose other components, excluding those belonging to the cassette. This results in increased volume and cost. Furthermore, such a structure forces the guidewire to emerge from the rotating assembly of the external, elongated medical device and enter the cassette in a parabolic arc. (For example, U-shaped) Since the elongated medical device is made of a deformable material, there is a possibility that the elongated medical device may become twisted during the rotation process. As a result, the rotation provided by the rotation assembly of the elongated medical device may not be properly transmitted to the elongated medical device. In addition, if the twist is excessive and the elongated medical device becomes entangled, the elongated medical device may become unusable. This may result in the useless consumption of the elongated medical device. In addition, a situation may arise where the elongated medical device exits the rotation assembly of the elongated medical device in the opposite direction from the direction in which it enters the cassette. As a result, the elongated medical device rotates within the cassette in the opposite direction from the direction of rotation of the rotation assembly of the elongated medical device. In this case, since the steering requires thinking in the opposite direction, precise steering may become difficult.

### [Detailed Description]

### [Problems to be solved]

Accordingly, the object of the present invention is to solve the aforementioned conventional problems, and to provide a rotation assembly, an elongated medical device movement assembly, and a medical robot using the same, which enable a user to precisely control an elongated medical device.

In addition, the present invention provides a rotation assembly, an elongated medical device movement assembly, and a medical robot using the same, which are not installed outside a cassette and can be installed inside a cassette.

In addition, the present invention provides a rotation assembly, an elongated medical device movement assembly, and a medical robot using the same, which are installed inside the cassette and thus can reduce costs while not requiring additional separate parts for installation outside the cassette.

In addition, the present invention provides a rotation assembly, an elongated medical device movement assembly, and a medical robot using the same, which are installed inside the cassette and thus simplify the structure and minimize errors during operation.

In addition, the present invention provides a rotation assembly, an elongated medical device movement assembly, and a medical robot using the same, in which as the elongated medical device passes only inside the cassette, the elongated medical device does not form a parabola such as a U shape outside the cassette, thereby preventing the elongated medical device from being twisted during rotation.

In addition, the present invention provides a rotation assembly, an elongated medical device movement assembly, and a medical robot using the same, which prevent the useless consumption of the elongated medical device by preventing twisting of the elongated medical device, thereby reducing costs and improving the completion of surgery.

In addition, the present invention provides a rotation assembly, an elongated medical device motion assembly, and a medical robot using the same, which can obtain the effect that the direction in which the elongated medical device is inserted into the elongated medical device motion assembly and the direction in which the elongated medical device is inserted into the opening of the shaft fixing part and passes through the support member and comes out through the opening of the plate spur gear are the same, regardless of the direction in which the elongated medical device enters from the outside of the cassette.

In addition, the present invention provides a rotation assembly, an elongated medical device motion assembly, and a medical robot using the same, wherein the rotational direction provided by the rotation assembly and the rotational direction of the elongated medical device are in the same direction with respect to the longitudinal central axis of the elongated medical device.

In addition, the present invention provides a rotation assembly, an elongated medical device motion assembly, and a medical robot using the same, which includes an offset member, thereby allowing a portion of the elongated medical device in contact with the offset member to be spaced apart by a predetermined distance d from an imaginary line extending along the rotational axis of the rotation member.

### [Means of solving problem]

An elongated medical device rotation assembly according to one aspect of the present invention for solving the above problem comprises a rotation member, a support member, and an offset member. The rotation member rotates by driving an actuator. The support member is coupled to the rotation member and supports at least a portion of the elongated medical device and rotates the elongated medical device about a longitudinal axis in conjunction with the rotation of the rotation member. The support member includes an offset member for spacing at least a portion of the elongated medical device from an imaginary line extending along the rotational axis of the rotation member by a predetermined distance d on a surface on which the elongated medical device is placed.

In the elongated medical device rotation assembly according to another aspect of the present invention, the rotation member includes an opening for inserting the elongated medical device. The support member includes an opening for inserting the elongated medical device. The predetermined distance d represents the maximum distance from an imaginary straight line connecting the opening of the rotation member and the opening of the support member to at least a portion of the elongated medical device.

In the elongated medical device rotation assembly according to another aspect of the present invention, the offset member includes a free roller that is axially supported in a direction perpendicular t to a surface on which the elongated medical device is placed. The free roller includes a contact surface, at least a portion of which abuts at least a portion of the elongated medical device.

Preferably, the free roller can freely rotate according to friction with the elongated medical device when the elongated medical device moves forward and backward in the longitudinal direction.

In the elongated medical device rotation assembly according to another aspect of the present invention, a groove may be formed on the contact surface of the offset member that is in contact with at least a portion of the elongated medical device.

The elongated medical device rotation assembly according to another aspect of the present invention includes at least two offset members.

In the elongated medical device rotation assembly according to another aspect of the present invention, the support member further includes a movement guide. The offset member may be installed to be movable along the movement guide.

A medical robot using the elongated medical device rotation assembly according to one aspect of the present invention comprises a frame, a track, a cassette platform, a forward/backward assembly, and a rotation assembly.

The track is coupled to the frame. The cassette platform is coupled to be movable along the track. The forward/backward assembly is provided on the cassette platform and is for forward/backward moving the elongated medical device. The elongated medical device rotation assembly is for rotating the elongated medical device. The elongated medical device rotation assembly includes a rotation member and a support member. The rotation member rotates by the driving of an actuator. The support member supports the elongated medical device and rotates the elongated medical device in conjunction with the rotation of the rotation member. The support member includes an offset member. The offset member offsets at least a portion of the elongated medical device from the rotational axis of the elongated medical device by an offset d on a surface on which the elongated medical device is placed.

In the medical robot using an elongated medical device according to another aspect of the present invention, the rotation member includes an opening for inserting the elongated medical device. The support member includes an opening for inserting the elongated medical device. The offset d represents the distance from a straight line connecting the opening of the rotation member and the opening of the support member to at least a portion of the elongated medical device.

The elongated medical device rotation assembly according to one aspect of the present invention comprises a rotation member, a support member, and a free roller. The rotation member rotates by driving an actuator. The support member supports the elongated medical device and rotates the elongated medical device about a longitudinal axis in conjunction with the rotation of the rotation member. The support member includes a free roller that is axially supported in a direction perpendicular to a surface on which the elongated medical device is placed.

In the elongated medical device rotation assembly according to another aspect of the present invention, the free roller includes a contact surface, at least a portion of which abuts at least a portion of the elongated medical device.

In the elongated medical device rotation assembly according to another aspect of the present invention, when the elongated medical device is placed on the support member, at least a portion of the elongated medical device is spaced apart from the central axis in the forward and backward direction of the elongated medical device by the free roller by a maximum distance d.

In the elongated medical device rotation assembly related to another aspect of the present invention, the free roller can freely rotate according to frictional force with the elongated medical device when the elongated medical device moves forward and backward in the longitudinal direction.

In the elongated medical device rotation assembly according to another aspect of the present invention, a groove may be formed on the contact surface of the free roller.

In the elongated medical device rotation assembly according to another aspect of the present invention, the free roller may include at least two free rollers.

The elongated medical device movement assembly according to one aspect of the present invention comprises a forward/backward assembly and the elongated medical device rotation assembly. The forward/backward assembly is for moving the elongated medical device forward and backward. The elongated medical device rotation assembly is for rotating the elongated medical device. The elongated medical device rotation assembly may include any one of the elongated medical device rotations related to another aspects of the present invention.

The medical robot related to according to one aspect of the present invention may include the elongated medical device movement assembly according to one aspect of the present invention.

### [Effect of Invention]

According to one embodiment of the present invention, the elongated medical device rotation assembly is located inside the cassette, thereby providing an advantage in having a simpler structure than when it is located outside the cassette.

In addition, there is the advantage of reducing the manufacturing cost of the elongated medical device rotation assembly and reducing the volume of the entire medical robotic system for rotating the elongated medical device.

Additionally, as the elongated medical device passes only within the cassette, there is no need for the elongated medical device to form a parabolic curve, such as a U-shape, outside the cassette, thereby providing an advantage of eliminating the possibility of the elongated medical device twisting during rotation.

Furthermore, by preventing the elongated medical device from twisting, unnecessary wear and tear can be prevented.

Furthermore, there is an advantage that the direction in which the elongated medical device enters the opening of the rotating member and the opening of the shaft fixing member, respectively, and the direction in which it exits the elongated medical device rotating assembly are aligned.

Additionally, the rotation assembly for the elongated medical device is provided that enables precise manipulation of the elongated medical device.

Furthermore, according to one embodiment of the present invention, the support member may include an offset member. Accordingly, when the elongated medical device rotates along with the support member, there is an advantage in facilitating rotation of the elongated medical device.

Furthermore, according to one embodiment of the present invention, the offset member may be provided with a groove, thereby providing an advantage of preventing the elongated medical device from being displaced in the y-axis or z-axis during rotation.

Furthermore, according to one embodiment of the present invention, a movement guide portion may be provided on the plate of the support member. This allows the offset member to move freely in the y-axis direction, thereby providing an advantage of easily mounting the elongated medical device to the elongated medical device rotation assembly.

### [Brief description of the drawing]

Figures 1a and 1b are schematic diagrams for explaining the forward movement and rotation of an elongated medical device according to the conventional art, respectively, and Figure 1c is an exploded perspective view of the pinch and forward assembly of the elongated medical device according to the conventional art. Figure 1d is an exploded perspective view of the elongated medical device rotation assembly according to the conventional art.
Figure 2 is a schematic diagram of an elongated medical device system (100) according to one embodiment of the present invention.
Figure 3(a) is a schematic diagram for explaining the operation of a forward/backward assembly (300) that moves an elongated medical device (800) forward/backward, and Figure 3(b) is a schematic diagram for explaining the operation of the elongated medical device rotation assembly (400) that rotates the elongated medical device (800).
Figure 4 is a schematic perspective view of a medical robot according to one embodiment of the present invention.
Figure 5(a) is a schematic plan view illustrating a state in which an elongated medical device movement assembly (200) according to one embodiment of the present invention rotates the elongated medical device (800), and Figure 5(b) is a schematic plan view illustrating a state in which the elongated medical device movement assembly (200) moves the elongated medical device (800) forward and backward.
Figure 6(a) is a front view of the portion i in Figure 5(a) of the elongated medical device movement assembly (200) according to one embodiment of the present invention, and Figure 6(b) is a rear view of the portion ii in Figure 5(a) of the elongated medical device movement assembly (200) according to one embodiment of the present invention.
Figure 7 is a detailed configuration diagram illustrating a state in which a support member (500) of the elongated medical device movement assembly (200) according to one embodiment of the present invention is rotating.
Figure 8 is a schematic plan view of the elongated medical device movement assembly (200) according to the second embodiment of the present invention.
Figure 9 is a detailed configuration diagram illustrating a state in which the support member (500) of the elongated medical device movement assembly (200) according to the second embodiment of the present invention, illustrated in Figure 8, is rotating.
Figure 10 is an exploded perspective view showing the offset member (660) being coupled to the elongated medical device support member (650) in the elongated medical device movement assembly (200) according to the second embodiment of the present invention.
Figure 11 is a drawing showing various embodiments of a groove (670) formed on a contact surface of an offset member (660) in the elongated medical device movement assembly (200) according to the second embodiment of the present invention.
Figure 12 is a drawing showing another embodiment of a plate (650) on which the offset member (660) is mounted in the elongated medical device movement assembly (200) according to the second embodiment of the present invention.
Figure 13 is a schematic plan view of the elongated medical device movement assembly (200) according to the third embodiment of the present invention.
Figure 14 is a detailed configuration diagram illustrating a state in which the support member (500) of the elongated medical device movement assembly (200) according to the third embodiment of the present invention, illustrated in Figure 13, is rotating.
Figure 15 is a detailed front view of the elongated medical device rotation assembly (400) according to the third embodiment of the present invention illustrated in Figure 13, along with left and right side views.
Figure 16 is a schematic plan view of the elongated medical device movement assembly (200) according to the fourth embodiment of the present invention.
Figure 17 is a detailed configuration diagram illustrating a state in which the support member (500) of the elongated medical device movement assembly (200) according to the fourth embodiment of the present invention illustrated in Figure 16 is rotating.
Figure 18 is a detailed perspective view illustrating a state in which the support member (500) of the elongated medical device movement assembly (200) according to the fourth embodiment of the present invention illustrated in Figure 16 is rotating.
Figure 19 is a detailed front view of the elongated medical device movement assembly (200) according to the fourth embodiment of the present invention, viewed from the lateral direction (-Y direction).
Figure 20 is a view illustrating various embodiments of the groove (670) formed on the contact surface of an offset member (660) in the elongated medical device movement assembly (200) according to the fourth embodiment of the present invention.
Figure 21(a) is a schematic plan view of the elongated medical device movement assembly (200) according to the fifth embodiment of the present invention, Figure 21(b) is a front view of portion i in Figure 21(a) of the elongated medical device movement assembly (200) according to the fifth embodiment of the present invention, and Figure 21(c) is a rear view of portion ii in Figure 21(a) of the elongated medical device movement assembly (200) according to the fifth embodiment of the present invention.
Figure 22(a) is a schematic plan view of the elongated medical device movement assembly (200) according to the sixth embodiment of the present invention, Figure 22(b) is a front view of portion i in Figure 22(a) of the elongated medical device movement assembly (200) according to the sixth embodiment of the present invention, and Figure 22(c) is a rear view of portion ii in Figure 22(a) of the elongated medical device movement assembly (200) according to the sixth embodiment of the present invention.
Figure 23(a) is a schematic plan view of the elongated medical device movement assembly (200) according to the seventh embodiment of the present invention, Figure 23(b) is a front view of portion i in Figure 23(a) of the elongated medical device movement assembly (200) according to the seventh embodiment of the present invention, and Figure 23(c) is a rear view of portion ii in Figure 23(a) of the elongated medical device motion assembly (200) according to the seventh embodiment of the present invention.
Figure 24(a) is a schematic plan view of the elongated medical device movement assembly (200) according to the eighth embodiment of the present invention, Figure 24(b) is a front view of portion i in Figure 24(a) of the elongated medical device movement assembly (200) according to the eighth embodiment of the present invention, and Figure 24(c) is a rear view of portion ii in Figure 24(a) of the elongated medical device movement assembly (200) according to the eighth embodiment of the present invention.
Figure 25(a) is a schematic plan view of the elongated medical device movement assembly (200) according to the ninth embodiment of the present invention, Figure 25(b) is a front view of portion i in Figure 25(a) of the elongated medical device movement assembly (200) according to the ninth embodiment of the present invention, and Figure 25(c) is a rear view of portion ii in Figure 25(a) of the elongated medical device movement assembly (200) according to the ninth embodiment of the present invention.
Figure 26(a) is a schematic plan view of the elongated medical device movement assembly (200) according to the tenth embodiment of the present invention, Figure 26(b) is a front view of portion i in Figure 26(a) of the elongated medical device movement assembly (200) according to the tenth embodiment of the present invention, and Figure 26(c) is a rear view of portion ii in Figure 26(a) of the elongated medical device movement assembly (200) according to the tenth embodiment of the present invention.
Figure 27(a) is a schematic plan view of the elongated medical device movement assembly (200) according to the eleventh embodiment of the present invention, Figure 27(b) is a front view of portion i in Figure 27(a) of the elongated medical device movement assembly (200) according to the eleventh embodiment of the present invention, and Figure 27(c) is a rear view of portion ii in Figure 27(a) of the elongated medical device movement assembly (200) according to the eleventh embodiment of the present invention.

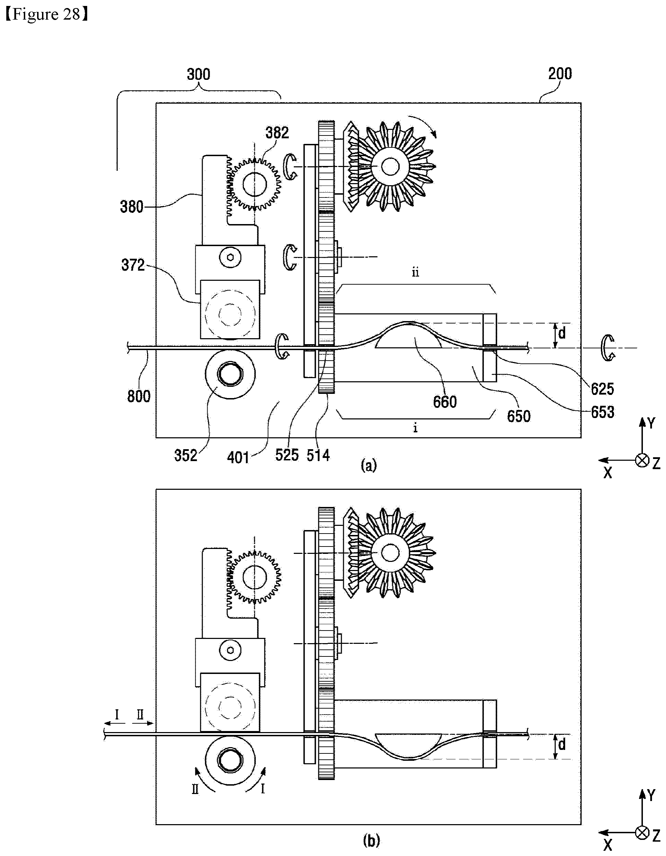

Figure 28 is a schematic plan view of the elongated medical device movement assembly (200) according to the twelfth embodiment of the present invention. Figure 28(a) is a schematic plan view of the elongated medical device movement assembly (200) according to the twelfth embodiment of the present invention, in which the offset member (660), for example, has a semicircular shape in the +y-axis direction and a protrusion protruding in the -z-axis direction, is placed on the support member (650). Figure 28(b) is a schematic plan view of the elongated medical device movement assembly (200) according to the twelfth embodiment of the present invention, in which the offset member (660), for example, has a semicircular shape in the -y-axis direction and a protrusion protruding in the -z-axis direction, is placed on the support member (650).
Figure 29(a) is a schematic plan view of the elongated medical device movement assembly (200) in a step of mounting the elongated medical device (800) on the elongated medical device rotation assembly (400) according to the thirteenth embodiment of the present invention. Figure 29(b) is a schematic plan view of the elongated medical device movement assembly (200) in a step of rotating the elongated medical device (800) after moving and fixing the offset member (665) in the +y-axis direction according to the thirteenth embodiment.

### [Best modes for carrying out the invention]

### Overall Structure

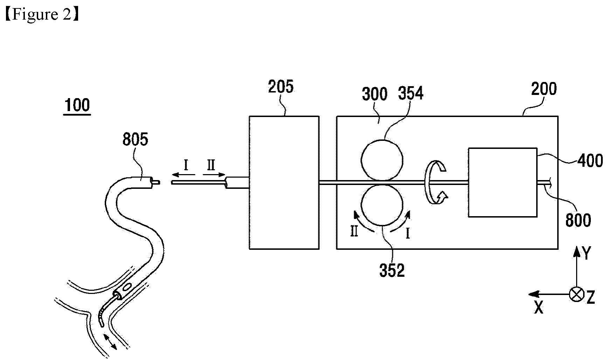

Figure 2 is a schematic diagram of the elongated medical device system (100) according to one embodiment of the present invention. Hereinafter, with reference to Figure 2, the forward movement and rotation of the elongated medical device (800) according to one embodiment of the present invention will be described.

The "elongated medical device" mentioned in this specification refers to a thin and long medical device that can be inserted into the body. Such an elongated medical device (800) may include, for example, an intravascular insertion device, a catheter, a guidewire, etc. In Figure 2, the direction in which the elongated medical device (800) moves forward is referred to as x-direction, the direction in which it moves backward is referred to as -x-direction, the upward direction in the drawing while being orthogonal to the x-direction (also referred to as the lateral direction of the elongated medical device movement assembly) is referred to as y-direction, and the direction orthogonal to the x-direction and the y-direction is referred to as z-direction.

As illustrated in Figure 2, the elongated medical device system (100) according to one embodiment of the present invention includes a movement assembly (200) for moving the elongated medical device (800) forward, backward, and rotating. Figure 2 illustrates an example in which the elongated medical device system (100) moves forward, backward, and rotates a large-diameter elongated medical device (805) having a large diameter, rather than the elongated medical device (800) into which the elongated medical device (800) is coaxially inserted, that is, two elongated medical devices in a coaxial insertion relationship. For example, the elongated medical device (800) may be a guidewire inserted into a blood vessel, and the large-diameter elongated medical device (805) may be a catheter inserted into a blood vessel.

According to one exemplary embodiment of the present invention of Figure 2, the elongated medical device system (100) may further include an elongated medical device movement assembly (200) for moving the elongated medical device (800) forward and backward and rotating, and a separate large-diameter elongated medical device movement assembly (205) for moving the large-diameter elongated medical device (805) forward and backward and rotating. According to one embodiment of the present invention, when the elongated medical device (800) is inserted into the large-diameter elongated medical device (805), the large-diameter elongated medical device movement assembly (205) moves the large-diameter elongated medical device (805) to a desired point within the body. Thereafter, when the movement of the large-diameter elongated medical device (805) has stopped, the elongated medical device movement assembly (200) further moves only the elongated medical device (800) within the body to reach the desired point.

Hereinafter, only the operation of the elongated medical device movement assembly (200) is described, but all or part of the structure and operation of the elongated medical device movement assembly (200) may be employed in the large-diameter elongated medical device movement assembly (205). In addition, although only two elongated medical device movement assemblies are illustrated in Figure 2, in the case of three or four elongated medical devices, three or four elongated medical device movement assemblies may be provided to move each elongated medical device forward and backward and rotate each elongated medical device.

The elongated medical device movement assembly (200) includes a forward/backward assembly (300) for moving the elongated medical device (800) forward/backward and an elongated medical device rotation assembly (400) for rotating the elongated medical device (800).

The forward/backward assembly (300) includes a roller assembly. The roller assembly includes a first roller (352) and a second roller (354). The first roller (352) and the second roller (354) are spaced apart from each other on the Y-axis. The elongated medical device (800) can be positioned between the first roller (352) and the second roller (354). The elongated medical device (800) can form contact surfaces with the first roller (352) and the second roller (354), respectively. When the first roller (352) and the second roller (354) rotate while contacting each other with the elongated medical device (800) interposed therebetween, the elongated medical device (800) can move forward or backward.

The elongated medical device rotation assembly (400) rotates the elongated medical device (800). The detailed structure and operation of the rotation assembly (400) will be described later.

Below, the structure and operation of the elongated medical device movement assembly (200) will be described. However, since the operation of the elongated medical device movement assembly (200) can also be applied to the large-diameter elongated medical device movement assembly (205), for the sake of convenience of explanation, only the operation of the elongated medical device movement assembly (200) will be described.

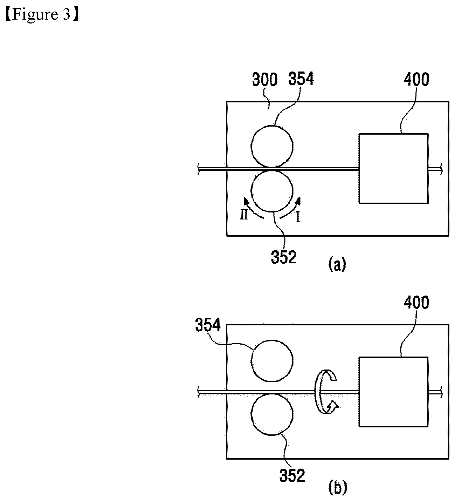

Figure 3 is a schematic diagram for explaining the operation of the forward/backward assembly (300) and the elongated medical device rotation assembly (400) in the elongated medical device movement assembly (200) to move the elongated medical device forward/backward and rotate the elongated medical device, respectively. Figure 3(a) is a schematic diagram for explaining the operation of the forward/backward assembly (300) to move the elongated medical device (800) forward/backward, and Figure 3(b) is a schematic diagram for explaining the operation of the elongated medical device rotation assembly (400) to rotate the elongated medical device (800).

As illustrated in Figure 3(a), in the forward/backward assembly (300), when the first roller (352) and the second roller (354) rotate while in contact with each other with the elongated medical device (800) interposed therebetween, the elongated medical device (800) can move forward or backward. As illustrated in Figure 3(a), when the first roller (352) rotates counterclockwise (direction I) and, at the same time, the second roller (354) rotates clockwise, the elongated medical device (800) moves forward (direction I). Conversely, when the first roller (352) rotates clockwise (direction II) and, at the same time, the second roller (354) rotates counterclockwise, the elongated medical device (800) moves backward (direction II).

As shown in Figure 3(b), in the forward/backward assembly (300), the elongated medical device rotation assembly (400) can rotate the elongated medical device (800) while the first roller (352) and the second roller (354) are spaced apart from each other with the elongated medical device (800) interposed therebetween.

### Overall configuration of a medical robot to which the present invention can be applied

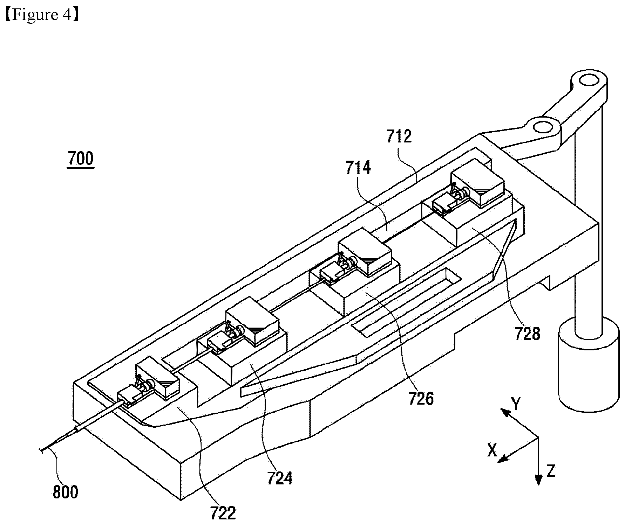

Figure 4(a) is a schematic perspective view of a medical robot according to one embodiment of the present invention.

As illustrated in Figure 4, the medical robot (700) according to one embodiment of the present invention includes a frame (712), a track (714), cassette platforms (722, 724, 726, 728), and a drive unit. Each cassette platform (722, 724, 726, 728) may include a drive unit within a housing. Each cassette may be mechanically attached to the cassette platform and the drive unit. In some examples, the cassette platform and the drive unit may be configured in multiple units.

The frame (712) is a support structure to which various components of the multi-axis catheter system are mechanically coupled and supported. The track (714) is located beneath and mechanically coupled to the frame (712). The track is mechanically coupled to the track (714) in a direction parallel to the longitudinal axis (e.g., in the X-direction) to allow translational movement of other components that can move along the track (714).

The cassette platforms (722, 724, 726, 728) may be mechanically coupled to the frame (712). The cassette platforms (722, 724, 726, 728) may include a cassette, a rotation assembly, and an forward assembly. In one embodiment, the cassette may be a disposable cassette. As illustrated in Figure 4, in one embodiment, the drive unit may be mechanically attached beneath the cassette.

As illustrated in Figure 4, in one embodiment, the cassette platform may be composed of four, including a proximal cassette platform (728), a first intermediate cassette platform (724), a second intermediate cassette platform (726), and a distal cassette platform (722). Each cassette platform is mechanically coupled to a respective drive unit to receive power.

The distal cassette platform (722) is fixed to the frame (712) and configured so as not to be translatable along a direction parallel to the longitudinal direction of the track by the drive unit, and cannot be translated along the track (714). The first and second intermediate cassette platforms (724, 726) and the proximal cassette platform (728) are attached to the frame (712) and configured so as to be translatable along a direction parallel to the longitudinal direction of the track (714) by the drive unit, and can be individually translatable along the track (714).

The elongated medical device movement assembly (200) according to one embodiment of the present invention can be assembled to each cassette platform (722, 724, 726, 728).

The embodiments described herein generally relate to systems and robots for endovascular procedures. More specifically, some of the embodiments described herein relate to a robot system for endovascular procedures and enable the operation of such a robot system. Figure 2 is a schematic diagram of a catheter and guidewire insertion medical robot to which the present invention can be applied.

In some examples, the catheter and guidewire insertion medical device comprises a catheter rotation assembly, a catheter advance assembly, a guidewire advance assembly, and a guidewire rotation assembly. Each assembly is independently mounted on the device and may be mounted within a single housing. Each assembly is independently movable. Each assembly generates translational and rotational movements of the catheter and guidewire. Through the translational and rotational movements, the distal ends of the catheter and guidewire can be steered.

The guidewire passes through the bore (inner diameter) of the catheter, which is larger than the outer diameter of the guidewire. The guidewire assembly and catheter assembly may each be composed of multiple or more units.

### Catheter Engagement and Advancement

During operation of the device, each cassette, along with its respective drive unit, is configured to move a respective catheter forward. The catheter moved forward by the cassette has its proximal end mechanically coupled to a Y-connector secured by the next more proximal cassette. For example, the catheter moved forward by the distal cassette has its proximal end mechanically coupled to a Y-connector secured by the first intermediate cassette, and the catheter moved forward by the first intermediate cassette has its proximal end mechanically coupled to a Y-connector secured by the second intermediate cassette. Thus, moving the catheter forward by the cassette can result in translation of the next more proximal cassette and its corresponding cassette platform and drive unit. The device may further include one or more translation assemblies capable of cooperatively translating the cassette (and corresponding cassette platform and drive unit) as the catheter having a proximal end to which the cassette is secured is moved forward, which may reduce or prevent catheter tensioning or bending. Additionally, the proximal cassette is configured to move the guidewire forward.

Additionally, each cassette is configured to rotate the respective catheter having a proximal end mechanically coupled to the cassette. The proximal cassette is also configured to rotate the guidewire.

In the device, each catheter and guidewire can move forward independently of each other. In addition, each catheter and guidewire can rotate independently of each other.

Each catheter may have different inner and outer diameters. For example, in the embodiment of Figure 34, a second catheter supported and driven by the second drive unit is moved forward through the bore (inner diameter) of the first catheter driven and supported by the first drive unit. A third catheter supported and driven by the third drive unit is moved forward through the bore (inner diameter) of the second catheter driven and supported by the second drive unit. The guidewire supported and driven by a fourth drive unit is moved forward through the bore (inner diameter) of a third catheter driven and supported by a third drive unit. Here, the inner diameter of the first catheter is larger than the outer diameter of the second catheter, and the inner diameter of the second catheter is larger than the outer diameter of the third catheter. Furthermore, the inner diameter of the third catheter is larger than the outer diameter of the guidewire.

The distal cassette is configured to move the first catheter forward together with the distal drive unit (first drive unit), and the first intermediate cassette is configured to rotate the first catheter together with the first intermediate drive unit (second drive unit). The first intermediate cassette is configured to move the second catheter forward together with the first drive unit (second drive unit), and the second intermediate cassette is configured to rotate the second catheter together with the second intermediate drive unit (third drive unit). The second intermediate cassette is configured to move the third catheter forward together with the second intermediate drive unit (third drive unit), and the proximal cassette is configured to rotate the third catheter together with the proximal drive unit (fourth drive unit). The proximal cassette is configured to rotate and move the guidewire forward.

The second intermediate cassette is configured to move the third catheter forward together with the second intermediate drive unit (third drive unit), and the proximal cassette is configured to rotate the third catheter together with the proximal drive unit (fourth drive unit). The proximal cassette is configured to rotate and move the guidewire forward.

The present invention relates to a medical robot and system for inserting elongated medical devices for endovascular procedures. The present invention may include a cassette system for inserting elongated medical devices (e.g., catheters and guidewires). The insertion of the elongated medical devices includes forward movement and rotation of the elongated medical devices. The cassette system includes a cassette, a frame, a track, a cassette platform, and a drive unit. The forward movement and rotation of the elongated medical devices in such a system may be independent of the forward movement or rotation of any other elongated medical devices. The present invention may include a casing or shroud around the frame. Each cassette may be a disposable cassette (e.g., suitable for a single endovascular procedure).

### [Modes for carrying out the invention]

### Overall structure of an elongated medical device movement assembly according to one embodiment of the present invention

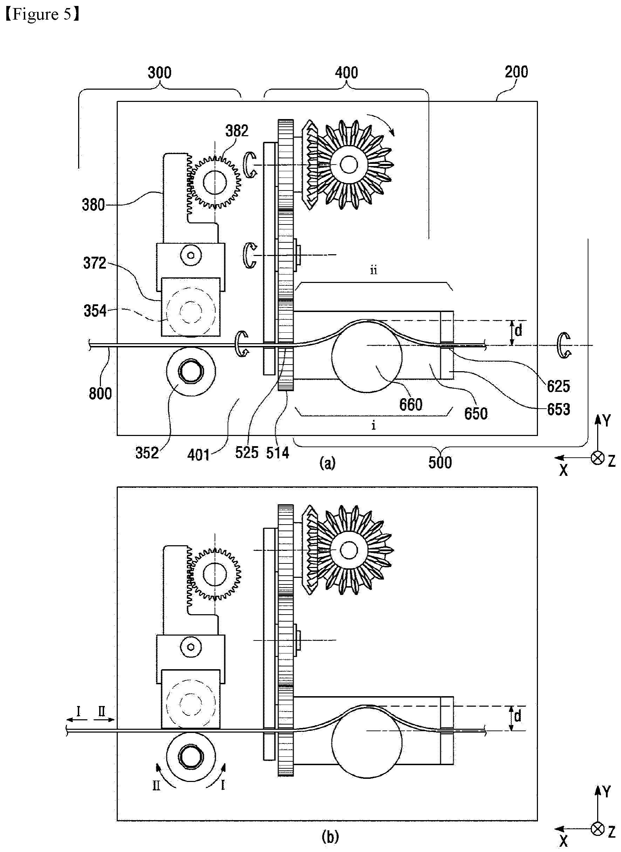

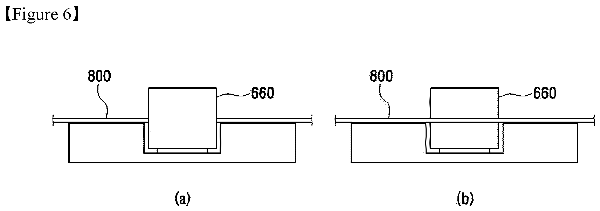

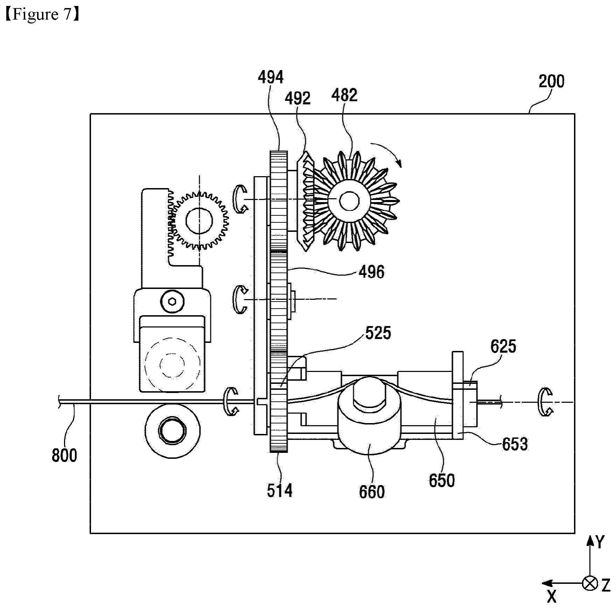

Figure 5 is a schematic plan view for explaining an elongated medical device movement assembly (200) according to one embodiment of the present invention. Figure 5(a) is a schematic plan view illustrating a state in which the elongated medical device movement assembly (200) according to one embodiment of the present invention rotates an elongated medical device (800), and Figure 5(b) is a schematic plan view illustrating a state in which the elongated medical device movement assembly (200) moves the elongated medical device (800) forward and backward. Figure 6(a) is a front view of portion i in Figure 5(a) of the elongated medical device movement assembly (200) according to one embodiment of the present invention. Figure 6(b) is a rear view of portion ii in Figure 5(a) of the elongated medical device movement assembly (200) according to one embodiment of the present invention. Figure 7 is a detailed configuration diagram showing a state in which a support member (500) of the elongated medical device movement assembly (200) according to one embodiment of the present invention is rotating.

As illustrated in Figure 5(a), the elongated medical device movement assembly (200) includes a forward/backward assembly (300) for moving the elongated medical device (800) forward/backward and an elongated medical device rotation assembly (400) for rotating the elongated medical device (800).

According to one exemplary embodiment of the present invention of Figures 5 to 7, the forward/backward assembly (300) includes a first roller (352) that rotates in a direction of moving the elongated medical device (800) forward and backward by frictional force when driven by an actuator (not shown), a second roller (354) that freely rotates by frictional force with the elongated medical device (800) moving forward and backward in accordance with the rotation of the first roller (352), a gear (382) that rotates when driven by the actuator (not shown), and a rack (380) that engages with the gear (382) to allow the second roller (354) to move laterally (Y-direction). As the gear (382) rotates, the rack (380) engages with the gear to move laterally, and the second roller (354) coupled to the rack (380) also moves laterally in conjunction with the rotation of the gear (382). According to one embodiment, an upper support frame (372) is coupled to the upper portion of the second roller (354), and the rack (380) and the upper support frame (372) can be connected by a connecting frame.

The elongated medical device rotation assembly (400) includes a rotation member (401) that rotates by the driving of an actuator (not shown), and an elongated medical device support member (500) that supports the elongated medical device (800) and rotates the elongated medical device (800) in conjunction with the rotation of the rotation member (401). At this time, the elongated medical device support member (500) is positionally adjusted and attached to the rotation member (401) so that the elongated medical device (800) can rotate about the longitudinal (X-direction) central axis of the elongated medical device (800). Furthermore, the elongated medical device (800) can be inserted to the center of the rotation axis of the rotation member (401) through the opening (525) so that the elongated medical device (800) can rotate about the same axis as the elongated (X-direction) central axis.

According to one embodiment of the present invention, the rotation member (401) may further include a bevel drive gear (482) that is connected to an actuator (not shown) and rotates by driving of the actuator (not shown), a bevel driven gear (492) that rotates by engaging vertically with the bevel drive gear (482), a first spur gear (494) that is fixed to the opposite surface of the teeth of the bevel driven gear (492) and rotates, a second spur gear (496) that rotates by engaging with the first spur gear (494), and a plate spur gear (514) that rotates by meshing with the second spur gear (496). Accordingly, the plate spur gear (514) rotates in conjunction with the driving of the actuator (not shown), and the rotational force of the plate spur gear (514) is transmitted to the elongated medical device (800) by the support member (500) described below, thereby causing the rotational movement of the elongated medical device (800). At this time, by aligning the central axis of the cross-section of the elongated medical device (800) with the rotational axis of the plate spur gear (514), the elongated medical device (800) can rotate about the same axis as the rotational axis of the plate spur gear (514). The gear connection structure from the actuator (not shown) to the plate spur gear (514) can be modified within a range conceivable by those skilled in the art. The transmission of power from the actuator (not shown) to the plate spur gear (514) can adopt a form other than a gear within a range conceivable by those skilled in the art.

According to one embodiment of the present invention, the rotation member (401) may further include a structure for inserting the elongated medical device (800). According to one exemplary embodiment of the present invention of Figures 5 to 7, an opening (525) for inserting the elongated medical device (800) into the plate spur gear (514) of the rotation member (401) may be formed to extend from the outer circumference of the plate spur gear (514) to the central axis. When a user wishes to move the elongated medical device (800) according to one embodiment of the present invention forward or backward or rotate it, the elongated medical device (800) may be inserted in advance through the opening (525) to the central axis of the plate spur gear (514). The opening (525) may be narrow and long and may have the shape of a slit extending from the outer circumference to the central axis.

According to one embodiment of the present invention, the elongated medical device support member (500) may further include an offset member (660) that is connected to the plate spur gear (514) at one side thereof and rotates about the same axis as the rotation of the plate spur gear (514), and is axially protruded in a direction perpendicular to a surface on which the elongated medical device (800) is placed on the support member (500). The offset member (660) is for spacing at least a portion of the elongated medical device (800) apart from an imaginary line extending along the rotational axis of the rotation member (401) by a predetermined distance d. The offset member (660) is installed at a position where at least a portion of its contact surface comes into contact with at least a portion of the elongated medical device (800) placed on the support member (500). Accordingly, when the elongated medical device (800) is placed on the support member (500), at least a portion of the elongated medical device (800) comes into contact with at least a portion of the contact surface of the offset member (660). In addition, the portion of the elongated medical device (800) that comes into contact with the offset member (660) is spaced apart from the central axis in the forward and backward direction of the elongated medical device (800) by a maximum distance d.

Specifically, the elongated medical device support member (500) may further include a plate (650) having one side connected to the plate spur gear (514) and rotating about the same axis as the rotation of the plate spur gear (514), a shaft fixing member (653) that supports the other side of the plate (650), and an offset member (660) formed on the upper surface of the plate (650) in a direction that protrudes perpendicularly to the upper surface. The plate (650) may rotate in conjunction with the rotation of the plate spur gear (514) by having one side connected to the plate spur gear (514) and the other side being supported by the shaft fixing member (653).

When the elongated medical device (800) moves forward and backward, frictional force may be generated at the part where the contact surfaces of the elongated medical device (800) and the offset member (660) come into contact with each other. The frictional force generated at the contact surface makes the rotation of the elongated medical device (800) more robust when the elongated medical device (800) rotates, while hindering the forward and backward movement of the elongated medical device (800) when the elongated medical device (800) moves forward and backward. Therefore, according to one embodiment of the invention, in order to reduce the frictional force generated between the contact surface of the offset member (660) and the elongated medical device (800), the offset member (660) may be formed as a free roller. Accordingly, the free roller (660) may freely rotate according to the frictional force between the contact surface and the elongated medical device (800) when the elongated medical device (800) in contact with the contact surface of the free roller moves forward and backward.

### Insertion of the elongated medical device (800) into the elongated medical device movement assembly (200)

First, the elongated medical device (800) is inserted into the elongated medical device movement assembly (200). When the elongated medical device (800) is inserted into the elongated medical device movement assembly (200) by the user, the elongated medical device (800) that has entered the space where the roller assembly is located is placed on the surfaces where the first roller (352) and the second rollers face each other. In addition, the elongated medical device (800) that has entered the rotation member (401) and the support member (500) is inserted into the central axes of the plate spur gear (514) and the shaft fixing member (653) through the openings (525) and (625) formed in the aforementioned plate spur gear (514) and the shaft fixing member (653), respectively.

The elongated medical device (800) that has entered a space in which the elongated medical device rotation assembly (400) is located by being inserted through the opening (525) and the opening (625) formed in the plate spur gear (514) and the shaft fixing member (653), respectively, is supported by the support member (500) on the same line as the rotation axis of the plate spur gear (514) that rotates around the x-axis. Here, among the elongated medical devices (800) supported by the support member (500), the elongated medical device (800) disposed near the offset member (660) comes into contact with the contact surface of the offset member (660) so that a frictional force is applied thereto.

### Forward and backward movement of the elongated medical device (800) by the elongated medical device movement assembly (200)

Hereinafter, with reference to Figures 5 to 7, the forward and backward movement of the elongated medical device (800) by the elongated medical device movement assembly (200) according to one embodiment of the present invention will be described.

When power is transmitted to the forward/backward drive assembly (not shown), and the gear (382) rotates by the driving of the actuator (not shown), the second roller (354) moves laterally (-Y direction) in conjunction with the rotation of the gear (382) to clamp the elongated medical device (800) together with the first roller (352) as shown in Figure 5(b).

When an actuator (not shown) for forward and backward movement of the elongated medical device (800) is driven, the power transmitted by the actuator (not shown) rotates the first roller (352). At this time, each roller has one surface that comes into contact with the elongated medical device (800). In addition, the first roller (352) and the second roller (354) are engaged with each other, and the second roller (354) rotates in the opposite direction according to the rotation of the first roller (352). At this time, the second roller (354) rotates freely. As a result, the elongated medical device (800) existing between the first roller (352) and the second roller (354) can move forward or backward in the x-axis direction.

As illustrated in Figure 5(b), when the first roller (352) rotates in the I direction (counterclockwise), the elongated medical device (800) moves forward in the X direction, and when the first roller (352) rotates in the II direction (clockwise), the elongated medical device (800) moves backward in the -X direction. As the elongated medical device moves forward in the X direction by the rotation of the first roller (352) of the roller assembly in the I direction and the rotation of the second roller opposite to the rotation, the end of the elongated medical device (800) can be supplied into the body. In addition, the rotation of the rollers opposite to the rotation can allow the elongated medical device (800) to be withdrawn from the body.

### Rotation movement of an elongated medical device (800) by an elongated medical device movement assembly (200)

Hereinafter, with reference to Figures 5 to 7, the rotation movement of the elongated medical device (800) by the elongated medical device motion assembly (200) according to one embodiment of the present invention will be described.

When power is transmitted to a rotation drive assembly (not shown), the power transmitted to the rotation drive assembly rotates a bevel drive gear (482). Accordingly, a bevel driven gear (492) that is vertically meshed with the bevel drive gear (482) rotates about the x-axis as its rotational axis. As the bevel driven gear (492) rotates, a first spur gear (494) that is shaft-fixed on the opposite surface to the bevel driven gear (492) also rotates about the x-axis as its rotational axis. At this time, a second spur gear (496) and a plate spur gear (514) that engage with the first spur gear (494) on the y-axis rotate according to the rotation of the first spur gear (494). At this time, the rotation of the first spur gear (494), the second spur gear (496) and the plate spur gear (514) causes the rotation of the elongated medical device (800) as described later.

As described above, the elongated medical device (800) that has entered the space where the elongated medical device rotation assembly (400) is inserted through the opening (525) and the opening (625) formed in the plate spur gear (514) and the shaft fixing member (653), respectively, is supported by the support member (500) on the same line as the rotation axis of the plate spur gear (514) that rotates around the x-axis.

Here, among the elongated medical devices (800) supported by the support member (500) as described above, the elongated medical devices (800) disposed near the offset member (660) are in contact with the contact surface of the offset member (660) so that frictional force is applied thereto. At this time, the elongated medical devices (800) may be supported by being in contact with a part or all of the contact surface of the offset member (660) on the support member (500). At this time, a tensile force is applied in the X-axis direction to the elongated medical device (800) extended in the X-axis direction, and accordingly, a force directed toward the center of the offset member (660) may be generated in the elongated medical device (800). This serves to make the rotation more robust when the elongated medical device (800) rotates by the rotation member (401) in the future. At this time, according to another embodiment of the present invention described later, a groove (670) may be dug in the contact surface of the offset member (660) so that the elongated medical device (800) can be guided therethrough. The elongated medical device (800) that passes through while surrounding around the offset member (660) passes through the opening (525) of the rotation member (401). At this time, the rotation of the rotation member (401) causes the elongated medical device (800) to rotate, and the rotation member (401) and the elongated medical device (800) rotate on the same axis.

In one embodiment shown in Figures 5 to 7, among the elongated medical devices (800) supported by the support member (500), the elongated medical device (800) positioned near the offset member (660) is supported so as to be in contact with a portion of the upper surface of the offset member (660).

According to the prior art as illustrated in Figure 1, the rotation of the forward shaft (9360) must cause the rotation of the forward spur gear (9356), which must be structured to cause the rotation of the forward spur gear (9358) and the forward shaft (9362) in the opposite rotational direction. Accordingly, the reverse rotation of the forward shafts (9360, 9362) causes the reverse rotation of the forward rollers (9352, 9354). That is, the forward shaft (9360) and the forward rollers (9352, 9354) can only rotate if the forward spur gear (9356) is present. In addition, the rotation assembly had to include a cap (9512), a collet (9516), a connector (9518), and a clamping bracket (9520). The plate spur gear (9514) had to be coupled to the cap (9512), and the cap (9512) had to include a threaded female connector. The connector (9518) had to include a threaded male connector (9522). Furthermore, when assembled, the threaded female connector of the cap (9512) had to engage with the threaded male connector (9522) of the connector (9518). Furthermore, when assembled, the collet (9516) had to be inserted into the tapered recess of the connector (9518), and the threaded female connector of the cap (9512) had to engage with the threaded male connector (9522) of the connector (9518). The clamping bracket (9520) had to hold the connector (9518) while it rotated. In this way, there was a problem that a separate part was required for the rotation assembly to exist externally to rotate the elongated medical device.

In contrast, as described above, according to the present invention, the rotation of the forward shaft (9362) can be directly transmitted to the forward rollers (9352, 9354) without requiring forward spur gears (9356, 9358).

Accordingly, the gap between the forward rollers (9352, 9354) becomes narrower. It is possible to prevent the elongated medical device (800) from not being able to move forward and backward and the forward rollers (9352, 9354) from spinning.

According to the present invention, the elongated medical device rotation assembly (400) can be embedded in the cassette rather than installed outside the cassette. This eliminates the need for additional components to surround the elongated medical device rotation assembly (400). This has a cost advantage. Furthermore, the elongated medical device rotation assembly (400) is configured as a component within the cassette, thereby simplifying the structure. This has the effect of minimizing errors during operation and also minimizing the overall volume of the medical robot.

In addition, by adopting the above structure, the present invention eliminates the need for the elongated medical device (800) to form a parabola, such as a U-shape, outside the medical robot, as the elongated medical device (800) passes only inside the cassette. As a result, the elongated medical device (800) can be prevented from being twisted during rotation. This has the effect of allowing the rotational force provided by the elongated medical device rotation assembly (400) to be directly transmitted to the elongated medical device (800).

In addition, as the elongated medical device (800) is not twisted, it is possible to prevent cases where the elongated medical device (800) is wasted unnecessarily, thereby reducing costs and improving the completion rate of the surgery.

In addition, since the elongated medical device rotation assembly (400) is located inside the cassette and the shaft fixing member (653) and the rotation member (401) are included in the elongated medical device rotation assembly (400), the direction in which the elongated medical device (800) enters the opening (525) of the rotation member (401) and the opening (625) of the shaft fixing member (653), respectively, and the direction in which it exits the elongated medical device rotation assembly (400) are all aligned. This enables the elongated medical device rotation assembly (400) and the elongated medical device (800) to rotate about the longitudinal central axis of the elongated medical device (800). As a result, a person controlling the medical robot to which the present invention is applied can control it more precisely.

In addition, according to one embodiment of the present invention, the offset member (660) may be added to the support member (500). Accordingly, when the elongated medical device (800) that enters through the opening (625) of the shaft fixing member (653) encounters the offset member (660) while proceeding toward the opening (525) of the plate spur gear (514), it passes while making contact with the contact surface of the offset member (660). At this time, a part of the elongated medical device (800) that makes contact with the offset member (660) is bent along an arc along the contact surface of the offset member (660). As a result, when the elongated medical device (800) rotates together with the support member (500) on which the elongated medical device (800) is placed, a torque is generated in an amount equal to the distance that the elongated medical device (800) is spaced apart from the central axis of the offset member (660). Accordingly, the rotation of the elongated medical device (800) is made easier than when the elongated medical device (800) simply passes through the plate (650) in a straight line. At this time, as in other embodiments described below, the number of the offset members may be multiple, and when the number of the offset members is plural, the offset members except for the offset members arranged in the middle may serve to hold the elongated medical device (800) so that it does not rise excessively. At this time, the plurality of offset members may exist on the same x-axis.

In addition, the offset members may have grooves (670) dug into the surfaces of the offset members so that the elongated medical device (800) can enter therein. The grooves (670) may be dug in a predetermined shape, for example, a rectangle, a triangle, a quadrilateral, or a U-shape, along the surfaces of the offset members. Accordingly, the elongated medical device (800) can completely enter the surfaces of the offset members. Accordingly, when the elongated medical device (800) enters the surfaces of the offset members, the lateral movement (Y-direction) of the elongated medical device (800) is restricted. This prevents the elongated medical device (800) from freely moving in the y-axis direction or the z-axis direction on the plate (650) when it rotates.

In addition, there may be a movement guide (611) that allows the offset member (660) to move freely in the y-axis direction on the plate (650). Through this, an advantage of being able to more easily mount the elongated medical device (800) on the elongated medical device rotation assembly (400) can be obtained. According to one embodiment of the invention, when mounting the elongated medical device (800) on the elongated medical device rotation assembly (400), the offset member (660) is mounted by moving downward along the movement guide (611) (-y-axis direction). And when rotating the elongated medical device (800), the offset member (660) can be fixed by moving upward along the movement guide (611) (+y-axis direction) and then rotated. Therefore, when mounting the elongated medical device (800) to the elongated medical device rotation assembly (400), the offset member (660) can be easily avoided. In this case, the movement of the offset member (660) along the movement guide (611) can be automated, of course. In one embodiment, the movement guide (611) may have a recess shape, a flat shape, or a protruding shape. In this case, the movement guide (611) may include a fixing member that can fix the offset member (660) to the corresponding position when the offset member (660) moves to the +y-side or -y-side end of the movement guide (611).

### Second embodiment of the present invention (embodiment in which a groove is formed on the contact surface of the offset member)

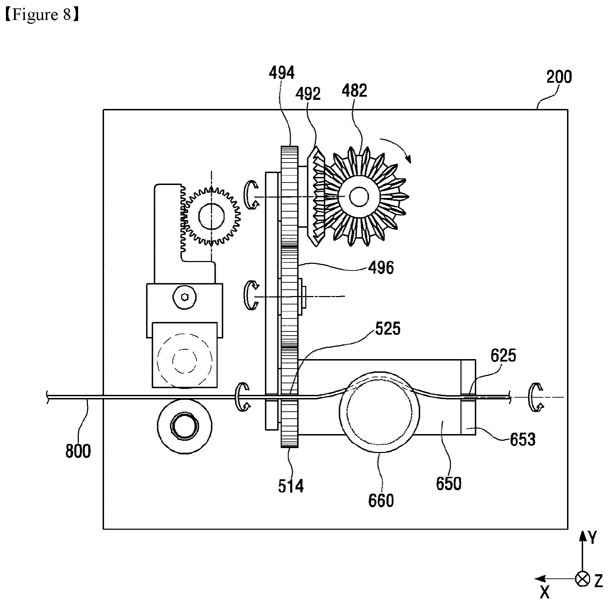

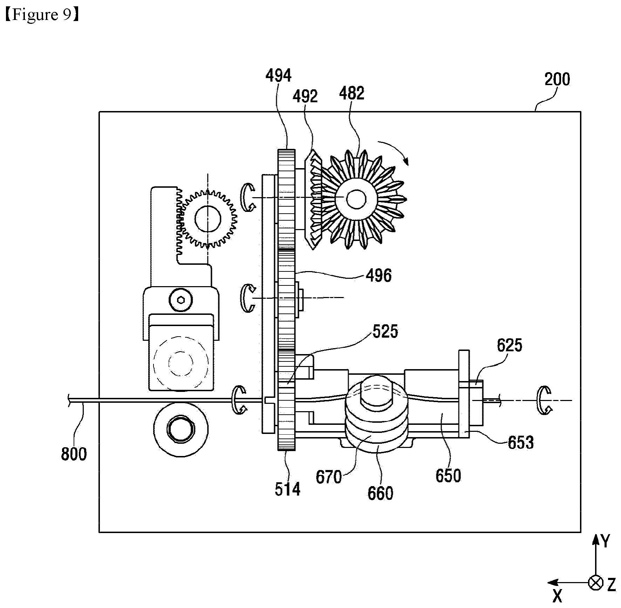

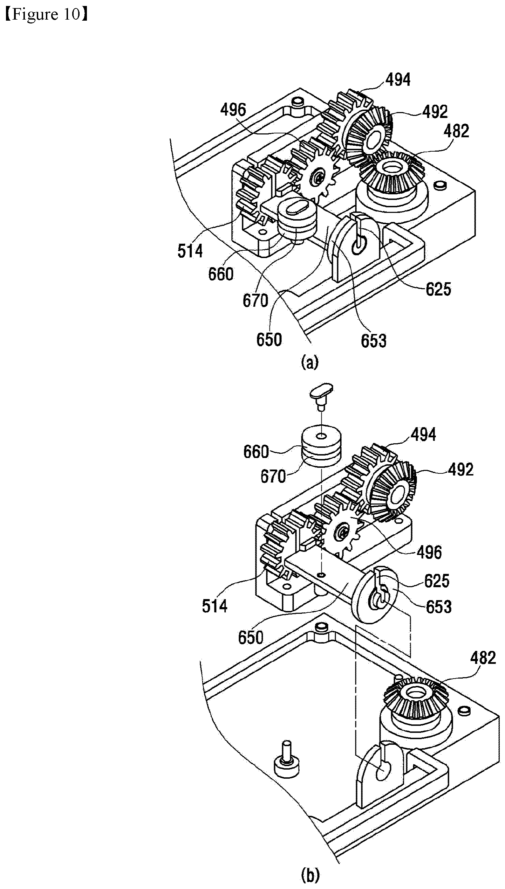

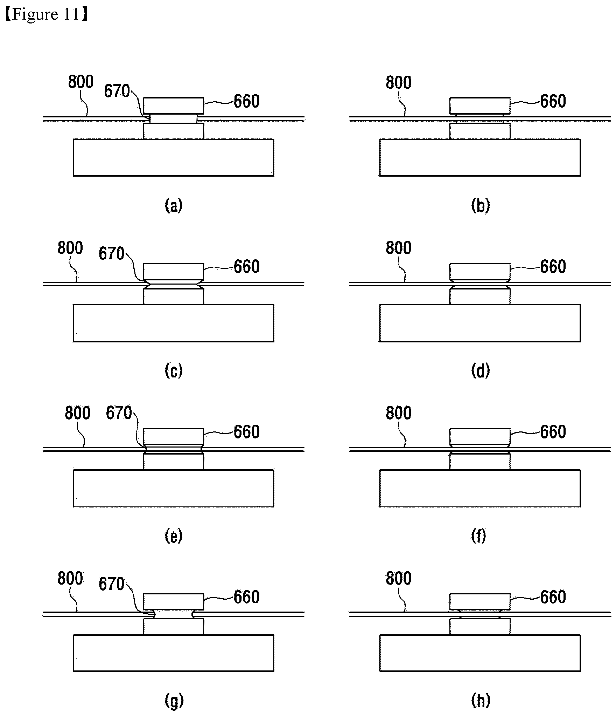

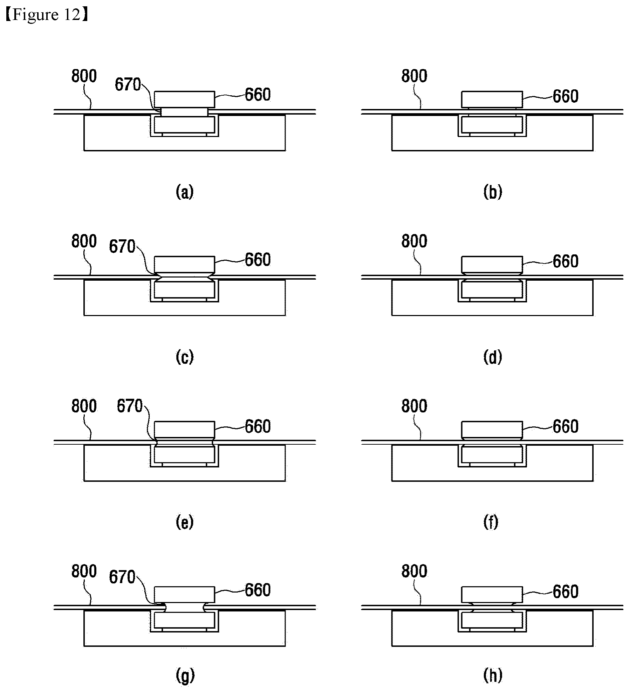

Figures 8 to 12 are views for explaining the elongated medical device movement assembly (200) according to the second embodiment of the present invention. Figure 8 is a schematic plan view of the elongated medical device movement assembly (200) according to the second embodiment of the present invention. Figure 9 is a detailed configuration diagram showing a state in which the support member (500) of the elongated medical device movement assembly (200) according to the second embodiment of the present invention, illustrated in Figure 8, is rotating. Figure 10 is an exploded perspective view showing the offset member (660) coupled to the elongated medical device support member (500) in the elongated medical device movement assembly (200) according to the second embodiment of the present invention. Figure 11 is a drawing showing various embodiments of the groove (670) formed on the contact surface of the offset member (660) in the elongated medical device movement assembly (200) according to the second embodiment of the present invention. Figure 12 is a drawing showing another embodiment of the plate (650) on which the offset member (660) is mounted in the elongated medical device movement assembly (200) according to the second embodiment of the present invention.

As illustrated in Figure 8, in the elongated medical device rotation assembly according to the second embodiment of the present invention, a U-shaped concave portion is formed on the upper surface of the plate (650) of the elongated medical device support member (500). The offset member (660) is disposed in the concave portion of the support member (500). The offset member (660) may have the groove (670) formed along the periphery of the contact surface (side surface). The groove (670) formed in the offset member (660) may have different cross-sections as illustrated in Figure 11, such as (a) and (b), (c) and (d), and (e) and (f). Of course, those skilled in the art may also employ other cross-sectional shapes. Furthermore, as illustrated in Figure 12, the offset member (660) may be disposed on the upper surface of the elongated medical device support member (500) rather than in the concave portion.

In this way, when the groove (670) is formed in the offset member (660), the elongated medical device (800) is connected to the offset member (660) by being wound along the groove (670), and when the roller (660) rotates freely, the elongated medical device (800) can be more securely placed within the groove (670). When the elongated medical device (800) can be more securely placed within the groove (670) of the offset member (660), the elongated medical device (800) can be more securely prevented from being separated from the offset member (660) when it rotates together with the elongated medical device support member (500).

The groove (670) may be dug along the surface of the offset member (660) in a predetermined shape, for example, a rectangle, a triangle, a quadrilateral, or a U-shape. Accordingly, the elongated medical device (800) can completely fit into the surface of the offset member (660). Accordingly, when the elongated medical device (800) fits into the surface of the offset member (660), the lateral movement (Y direction) of the elongated medical device (800) is restricted. This prevents the elongated medical device (800) from being detached from the offset member (660) as it rotates.

### Third embodiment of the present invention (embodiment having multiple offset members)

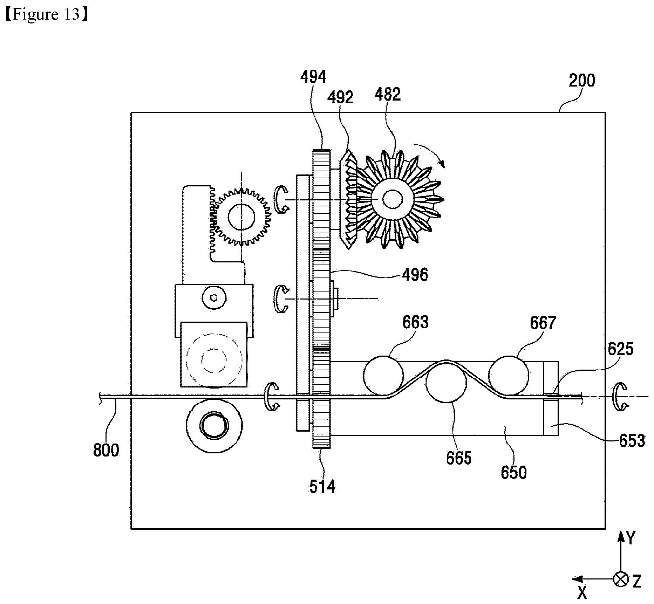

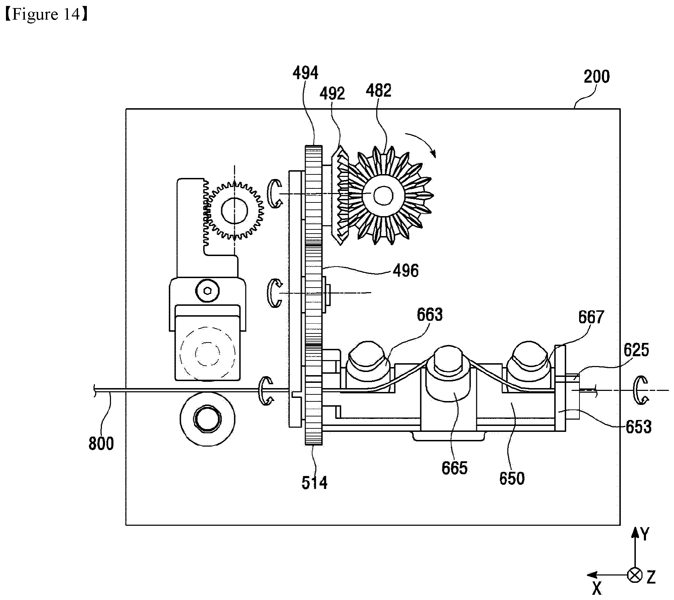

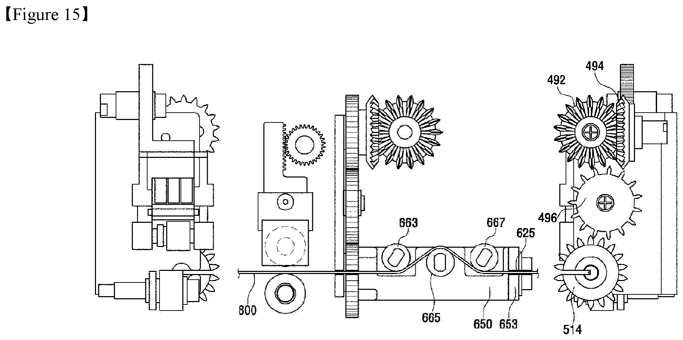

Figures 13 to 15 are views for explaining the elongated medical device movement assembly (200) according to the third embodiment of the present invention. Figure 13 is a schematic plan view of the elongated medical device movement assembly (200) according to the third embodiment of the present invention. Figure 14 is a detailed configuration diagram showing a state in which the support member (500) of the elongated medical device movement assembly (200) according to the third embodiment of the present invention illustrated in Figures 913 is rotating. Figure 15 is a drawing showing a detailed front view of the elongated medical device rotation assembly (400) according to the third embodiment of the present invention illustrated in Figure 13, along with left and right side views thereof.

As illustrated in Figures 13 to 15, the offset members (663, 665, 667) disposed on the elongated medical device support member (500) in the elongated medical device rotation assembly according to the third embodiment of the present invention may be configured in multiple numbers. The drawings illustrate a case of three members as an example. As illustrated in Figures 13, these three offset members (663, 665, 667) are arranged with their centers spaced apart in the y direction from the rotation axis of the elongated medical device (800), and furthermore, the first offset member (663) and the third offset member (667) are arranged with their centers spaced apart in the y direction by the same distance from the rotation axis of the elongated medical device (800), and the second offset member (665) is arranged with its center spaced apart by a shorter distance than the first offset member (663) and the third offset member (667), and these three rollers (663, 665, 657667) are arranged so that their outer shapes overlap when viewed in the x-axis direction. Furthermore, of course, the arrangement structure of these multiple rollers can be modified by those skilled in the art.

According to the third embodiment of the present invention, the elongated medical device rotation assembly configured as described above is configured such that the elongated medical device (800) is pulled apart by a certain portion from its rotational axis by a plurality of the offset members (663, 665, 657, 667) and hung.

When the elongated medical device (800) is pulled apart by a certain portion from its rotational axis and hung, the frictional force between the elongated medical device (800) and the roller increases, thereby more reliably preventing the elongated medical device (800) from being separated from the offset members (663, 665, 667) when rotating together with the elongated medical device support member (500). Furthermore, when there are multiple offset members, the offset members (663, 667), excluding the offset members positioned in the middle, can serve to hold the elongated medical device (800) so that it does not rise excessively. At this time, multiple rollers may exist on the same x-axis.

### Fourth embodiment of the present invention (embodiment in which there are a plurality of offset members and a groove is formed on the contact surface of each offset member)

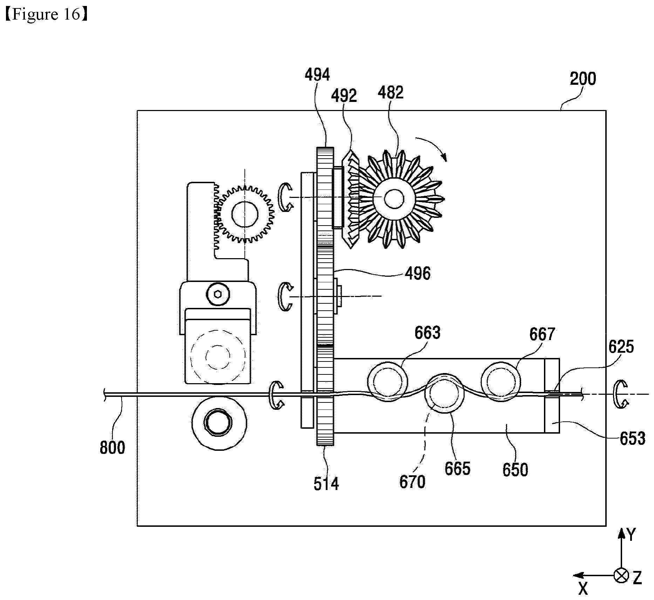

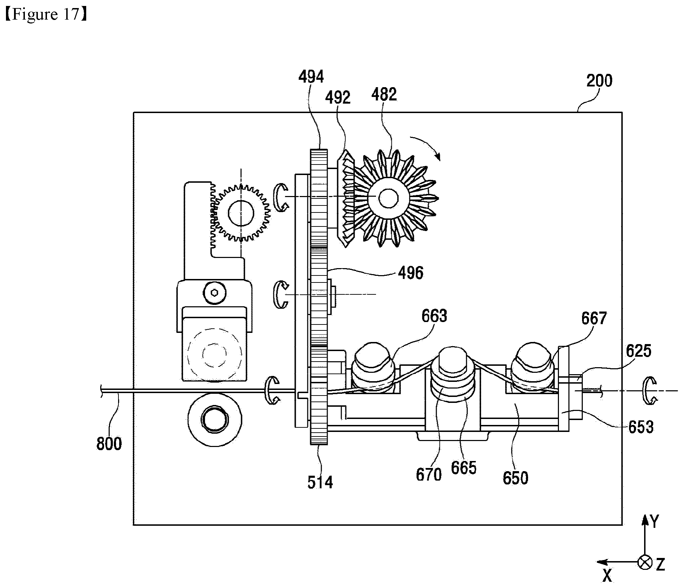

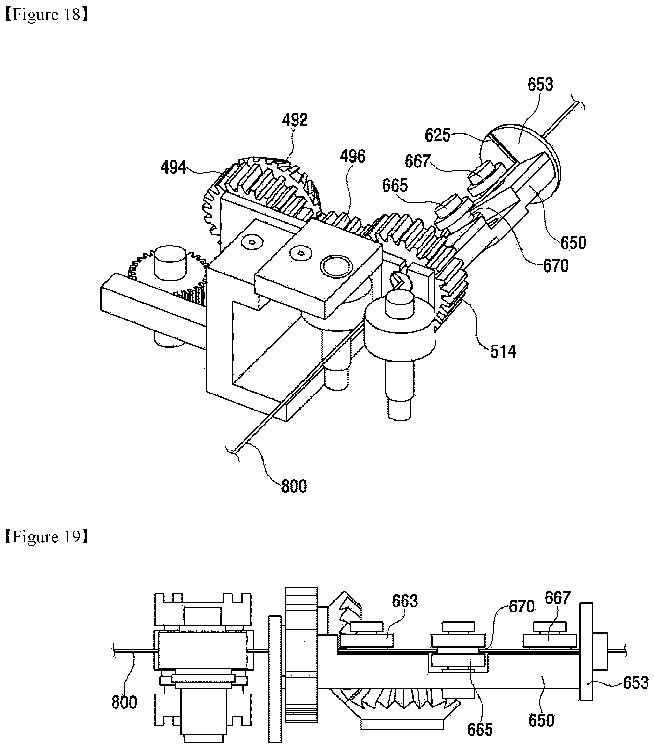

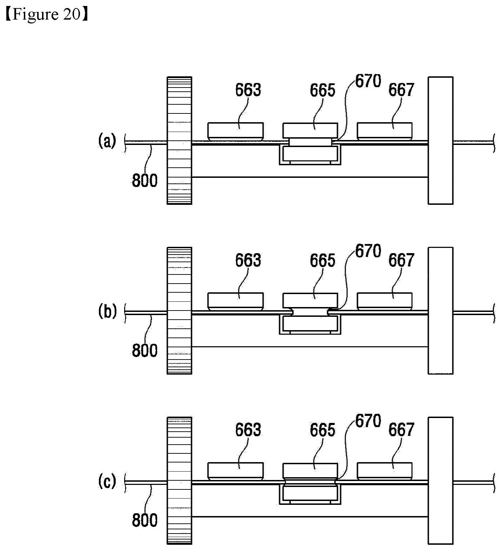

Figures 16 to 20 are drawings for explaining the elongated medical device movement assembly (200) according to the fourth embodiment of the present invention. Figure 16 is a schematic plan view of the elongated medical device movement assembly (200) according to the fourth embodiment of the present invention. Figure 17 is a detailed configuration diagram illustrating a state in which the support member (500) of the elongated medical device movement assembly (200) according to the fourth embodiment of the present invention illustrated in Figure 16 is rotating. Figure 18 is a detailed perspective view illustrating a state in which the support member (500) of the elongated medical device movement assembly (200) according to the fourth embodiment of the present invention illustrated in Figure 16 is rotating. Figure 19 is a detailed front view of the elongated medical device movement assembly (200) according to the fourth embodiment of the present invention as viewed from the side (-Y direction). Figure 20 is a drawing showing various embodiments of the groove (670) formed on the contact surface of the offset member (660) in the elongated medical device movement assembly (200) according to the fourth embodiment of the present invention.

As illustrated in Figures 16 to 19, in the elongated medical device movement assembly (200) according to the fourth embodiment of the present invention, a plurality of offset members (663, 665, 667) are provided, like the elongated medical device movement assembly (200) according to the third embodiment, and the groove (670) is formed along the periphery of the contact surface of each of the offset members (663, 665, 667). As illustrated in Figure 20, in the case where the groove (670) is formed along the periphery of the contact surface of the plurality of offset members (663, 665, 667), the cross-section of the groove (670) may be changed into the shapes of (a), (b), and (c), respectively.

In this case, when the groove (670) is formed in the offset member (663, 665, 667), when the elongated medical device (800) is connected to the offset member (663, 665, 667), it is connected by being wound along the groove (670), and when the offset member (663, 665, 667) rotates freely, the elongated medical device (800) can be more securely placed within the groove (670).

The groove (670) may be dug in a predetermined shape, for example, a rectangle, a triangle, a quadrilateral, or a U-shape, along the surface of the offset member (663, 665, 667). Accordingly, the guidewire can completely enter the surface of the offset member (663, 665, 667). Accordingly, when the elongated medical device (800) enters the surface of the offset member (663, 665, 667), the lateral movement (Y direction) of the elongated medical device (800) is restricted. This can prevent the elongated medical device (800) from being detached from the offset member (663, 665, 667) as it rotates.

In this way, when the elongated medical device (800) can be more securely placed in the groove (670) of the offset member (663, 665, 667), the elongated medical device (800) can be more securely prevented from being detached from the offset member (663, 665, 667) when rotating together with the elongated medical device support member (500).

### Fifth embodiment of the present invention (embodiment in which an elongated medical device is wound around an offset member by one or more turns)

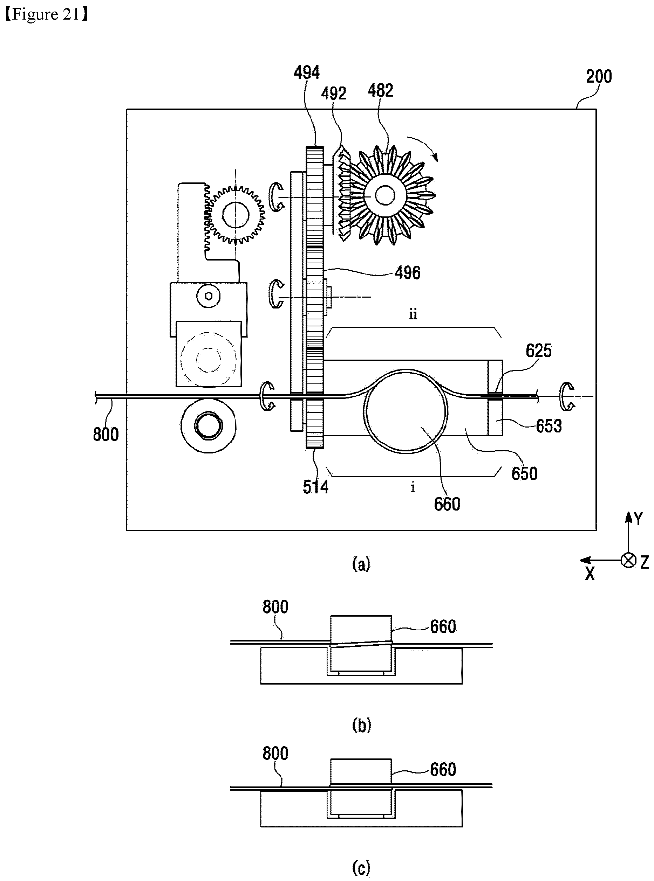

Figure 21(a) is a schematic plan view of the elongated medical device motion assembly (200) according to the fifth embodiment of the present invention. Figure 21(b) is a front view of portion i in Figure 21(a) of the elongated medical device movement assembly (200) according to the fifth embodiment of the present invention. Figure 21(c) is a rear view of portion ii in Figure 21(a) of the elongated medical device movement assembly (200) according to a fifth embodiment of the present invention.

As illustrated in Figure 21, in the elongated medical device rotation assembly according to the fifth embodiment of the present invention, the elongated medical device (800) can be connected by being wound around the offset member (660) by one rotation.

In this way, when the elongated medical device (800) is connected by being wound around the offset member (660) by one rotation, the frictional force between the elongated medical device (800) and the offset member (660) increases, thereby more reliably preventing the elongated medical device (800) from being separated from the offset member (660) when rotating together with the elongated medical device support member (500).

### Sixth embodiment of the present invention

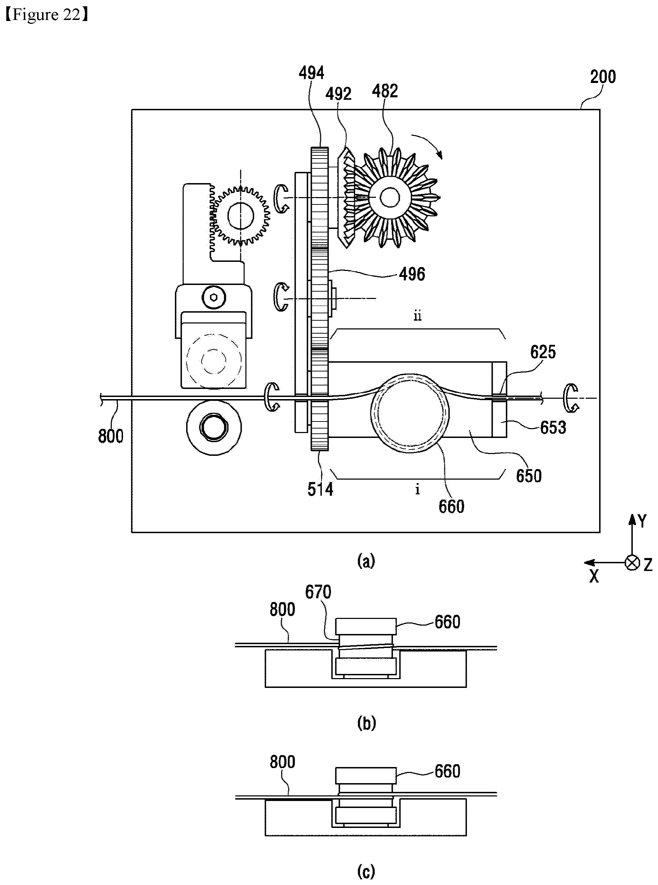

Figure 22(a) is a schematic plan view of the elongated medical device movement assembly (200) according to the sixth embodiment of the present invention. Figure 22(b) is a front view of portion i in Figure 22(a) of the elongated medical device movement assembly (200) according to the sixth embodiment of the present invention.

Figure 22(c) is a rear view of portion ii in Figure 22(a) of the elongated medical device movement assembly (200) according to the sixth embodiment of the present invention.

As illustrated in Figure 22, when the groove (670) is formed in the roller (660), the elongated medical device (800) can be connected by being wound around the offset member (660) by one rotation. In this case, it is possible to more reliably prevent the elongated medical device (800) from being separated from the offset member (660) when rotating together with the elongated medical device support member (500).

### Seventh embodiment of the present invention

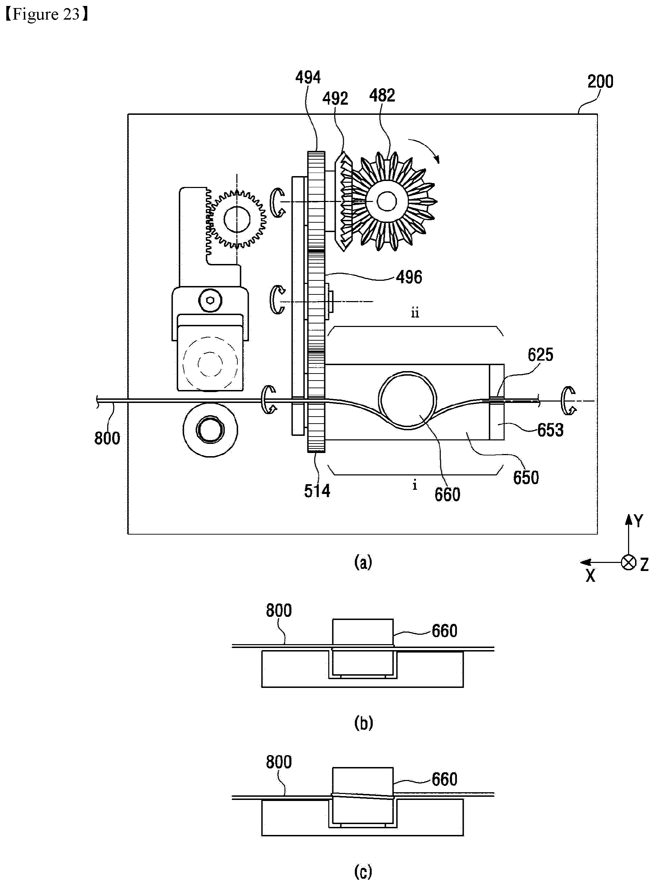

Figure 23(a) is a schematic plan view of the elongated medical device movement assembly (200) according to the seventh embodiment of the present invention. Figure 23(b) is a front view of portion i in Figure 23(a) of the elongated medical device movement assembly (200) according to the seventh embodiment of the present invention. Figure 23(c) is a rear view of portion ii in Figure 23(a) of the elongated medical device movement assembly (200) according to the seventh embodiment of the present invention.

As illustrated in Figure 23, the elongated medical device (800) may be connected by entering the lower portion of the offset member (660) and being wound around its circumference once. In this case, the elongated medical device (660800) can be more reliably prevented from being separated from the offset member (660) when rotating together with the elongated medical device support member (500).

### Eighth embodiment of the present invention

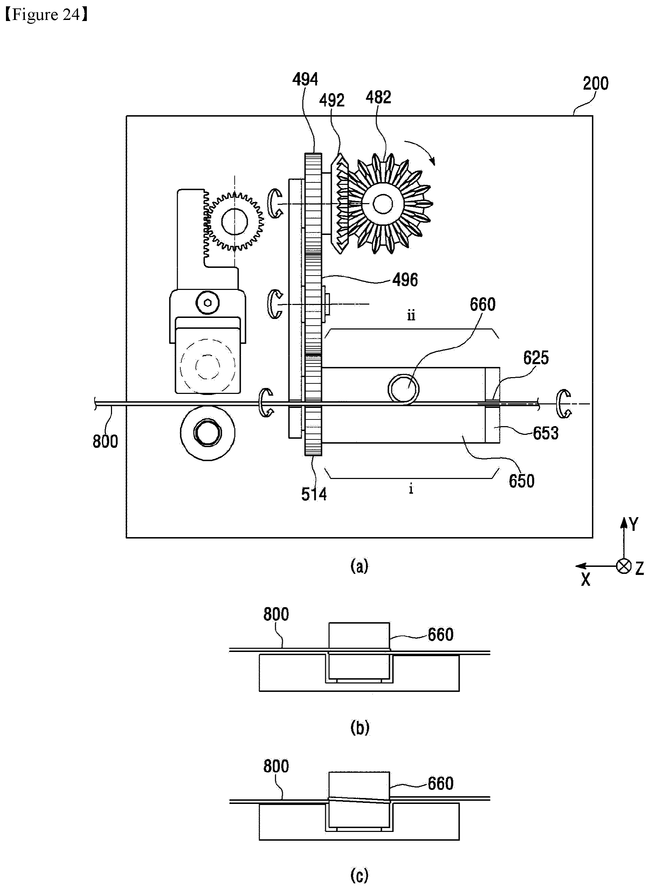

Figure 24(a) is a schematic plan view of the elongated medical device movement assembly (200) according to the eighth embodiment of the present invention. Figure 24(b) is a front view of portion i in Figure 24(a) of the elongated medical device movement assembly (200) according to the eighth embodiment of the present invention. Figure 24(c) is a rear view of portion ii in Figure 24(a) of the elongated medical device movement assembly (200) according to the eighth embodiment of the present invention.

As illustrated in Figure 24, when the roller (660) is positioned so that its lower circumference is in contact with the rotational axis of the elongated medical device (800), the elongated medical device (800) enters the lower portion of the offset member (660) and is connected by being wound around its circumference once. In this case, when the elongated medical device (660800) rotates together with the elongated medical device support member (500), it is possible to more reliably prevent the elongated medical device (800) from being separated from the offset member (660).

### Ninth embodiment of the present invention

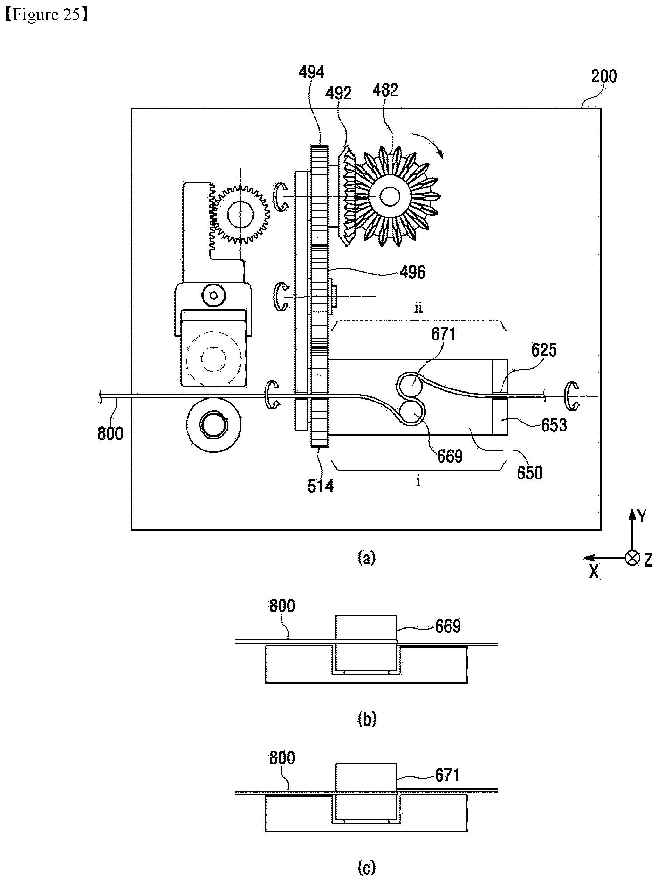

Figure 25(a) is a schematic plan view of the elongated medical device movement assembly (200) according to the ninth embodiment of the present invention. Figure 25(b) is a front view of portion i in Figure 25(a) of the elongated medical device movement assembly (200) according to the ninth embodiment of the present invention. Figure 25(c) is a rear view of portion ii in Figure 25(a) of the elongated medical device movement assembly (200) according to the ninth embodiment of the present invention.

As illustrated in Figure 25, according to the elongated medical device rotation assembly according to the ninth embodiment of the present invention, the offset members (669, 671) arranged on the elongated medical device support member (500) can be arranged coaxially and vertically side by side along the y-axis direction. In this case, the elongated medical device (800) enters while being wrapped around the upper end of the upper offset member (671), passes while being wrapped between the upper offset member (671) and the lower offset member (669), and is connected while surrounding around the lower end of the lower offset member (669), resulting in being connected while surrounding around the roller in an S shape.

When the elongated medical device (800) is connected in an S shape around the circumference of two offset members (669, 671), the frictional force between the elongated medical device (800) and the rollers (669, 671) increases, so that the elongated medical device (800) can be more reliably prevented from being separated from the rollers (669, 671) when rotating together with the elongated medical device support member (500).

### Tenth embodiment of the present invention

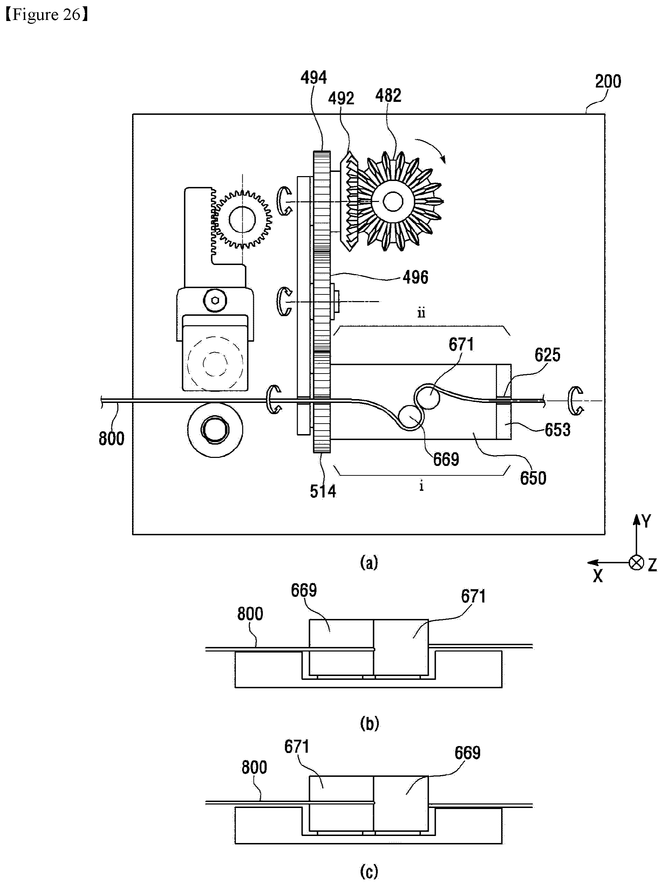

Figure 26(a) is a schematic plan view of the elongated medical device movement assembly (200) according to the tenth embodiment of the present invention. Figure 26(b) is a front view of portion i in Figure 26(a) of the elongated medical device movement assembly (200) according to the tenth embodiment of the present invention. Figure 26(c) is a rear view of portion ii in Figure 26(a) of the elongated medical device movement assembly (200) according to the tenth embodiment of the present invention.

In the tenth embodiment illustrated in Figure 26, in the embodiment illustrated in Figure 25, the upper offset member (671) and the lower offset member (669) are arranged spaced apart in the x-axis direction within a range in which their centers overlap along the y-axis direction. When the elongated medical device (800) is connected in an inclined S-shape around the circumference of the two offset members (669, 671), the frictional force between the elongated medical device (800) and the rollers (669, 671) increases, so that the elongated medical device (800) can be more reliably prevented from being separated from the rollers (669, 671) when rotating together with the elongated medical device support member (500).

Furthermore, although Figures 25 and 26 show that the elongated medical device (800) enters the upper side of the upper roller, it is of course also possible that it enters the lower side of the lower roller.

### Eleventh embodiment of the present invention

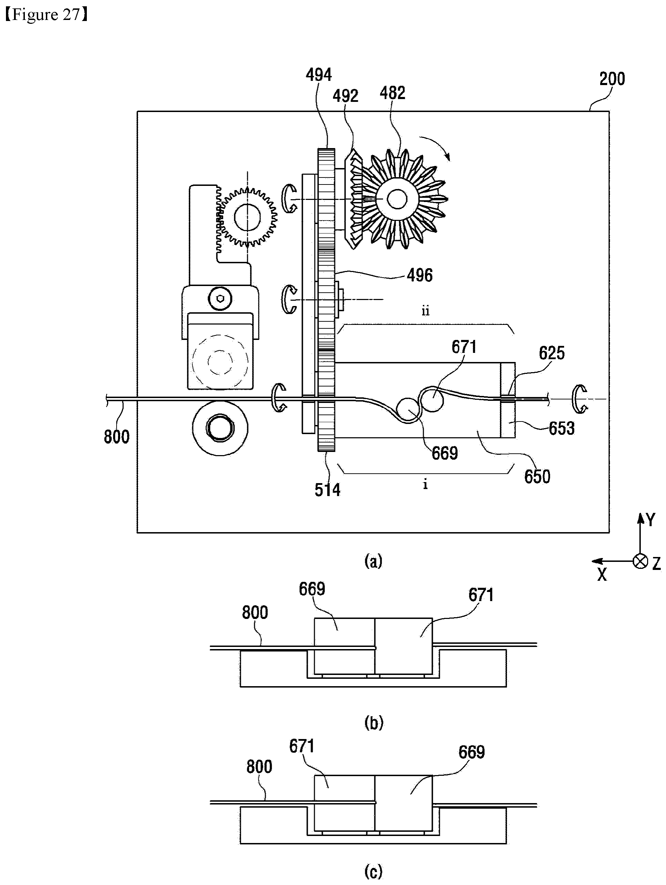

Figure 27(a) is a schematic plan view of the elongated medical device movement assembly (200) according to the eleventh embodiment of the present invention. Figure 27(b) is a front view of portion i in Figure 27(a) of the elongated medical device movement assembly (200) according to the eleventh embodiment of the present invention. Figure 27(c) is a rear view of portion ii in Figure 27(a) of the elongated medical device movement assembly (200) according to the eleventh embodiment of the present invention.

In the eleventh embodiment illustrated in Figure 27, in the embodiment illustrated in Figure 25, the upper offset member (671) and the lower offset member (669) can be arranged side by side along the x-axis within a range in which their centers overlap along the y-axis direction. In this case, there may additionally be a case in which they are arranged side by side coaxially along the x-axis without overlapping along the y-axis direction. When the elongated medical device (800) is wound and connected in an S-shape lying along the circumference of the two offset members (669, 671), the frictional force between the elongated medical device (800) and the rollers (669, 671) increases, so that the elongated medical device (800) can be more reliably prevented from being separated from the rollers (669, 671) when it rotates together with the elongated medical device support member (500).

### Twelfth embodiment of the present invention

Figure 28 is a schematic plan view of the elongated medical device movement assembly (200) according to the twelfth embodiment of the present invention. Figure 28(a) is a schematic plan view of the elongated medical device movement assembly (200) according to the twelfth embodiment of the present invention, in which the offset member (660), for example, has a semicircular shape in the +y-axis direction and a protrusion protruding in the -z-axis direction, is placed on the support member (650). Figure 28(b) is a schematic plan view of the elongated medical device movement assembly (200) according to the twelfth embodiment of the present invention, in which the offset member (660), for example, has a semicircular shape in the -y-axis direction and a protrusion protruding in the -z-axis direction, is placed on the support member (650). According to this embodiment, the elongated medical device (800) can be spaced apart by a predetermined distance d from an imaginary line extending along the rotational axis of the rotation member by the offset member (660). Accordingly, when the elongated medical device (800) rotates together with the support member (500) on which the elongated medical device (800) is placed, a torque is generated corresponding to the distance that the elongated medical device (800) is spaced apart from the central axis of the offset member (660). Accordingly, the rotation of the elongated medical device (800) is facilitated compared to when the elongated medical device (800) simply passes through the plate (650) in a straight line.

In this case, the shape of the protrusion is a semicircle as an example for explaining the invention, but any shape that can space the elongated medical device (800) in the +y or -y direction is not relevant.

### Thirteenth embodiment of the present invention

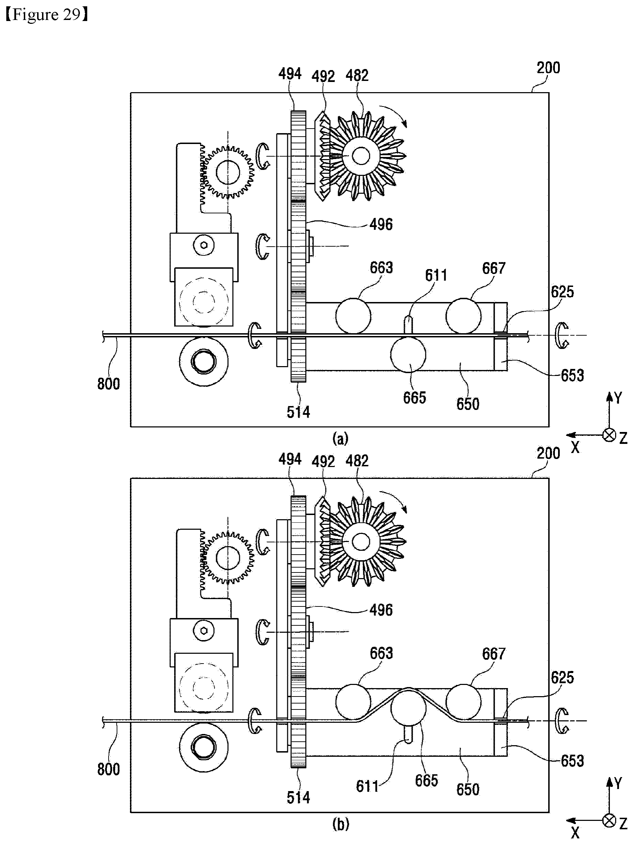

Figure 29(a) is a schematic plan view of the elongated medical device movement assembly (200) in a step of mounting the elongated medical device (800) to the elongated medical device rotation assembly (400) according to the thirteenth embodiment of the present invention. Figure 29(b) is a schematic plan view of the elongated medical device movement assembly (200) in a step of rotating the elongated medical device (800) after moving and fixing an offset member (665) in the +y-axis direction according to the thirteenth embodiment.

As illustrated in Figure 29(a), the elongated medical device movement assembly (200) according to the thirteenth embodiment of the present invention can have a movement guide (611) installed on the plate (650) on which the second offset member (665) is arranged. At this time, the movement guide (611) can facilitate movement of the second offset member (665) in the y-direction, thereby making it convenient to mount the elongated medical device (800) on the elongated medical device rotation assembly (400).

According to one embodiment of the invention, when mounting the elongated medical device (800) on the elongated medical device rotation assembly (400), the offset member (665) is mounted by moving downward (in the -y-axis direction) along the movement guide (611). Then, when rotating the elongated medical device (800), the offset member (665) can be fixed by moving upward (in the +y-axis direction) along the movement guide (611) and then rotated. Therefore, when mounting the elongated medical device (800) on the elongated medical device rotation assembly (400), the offset member (665) can be easily avoided. In this case, of course, that the movement of the offset member (665) along the movement guide (611) can be automated.

Electronic devices according to the various embodiments disclosed herein may take various forms. Electronic devices may include, for example, portable communication devices (e.g., smartphones), computer devices, portable multimedia devices, portable medical devices, cameras, wearable devices, or home appliances. Electronic devices according to the embodiments of this document are not limited to the aforementioned devices.

While the embodiments of the present invention have been described in detail above, the scope of the present invention is not limited thereto. Various modifications and improvements made by those skilled in the art utilizing the basic concepts of the present invention defined in the following claims also fall within the scope of the present invention.

### [Explanation of symbols]

100: elongated medical device system
800: elongated medical device
200: elongated medical device movement assembly
300: forward/backward assembly
400: elongated medical device rotation assembly
401: rotation member
500: support member
482: bevel drive gear
492: bevel driven gear
494: first spur gear
496: second spur gear
514: plate spur gear
525: opening
650: plate
611: movement guide
653: shaft fixing member
625: opening
660: offset member
663: first offset member
665: second offset member
667: third offset member
670: groove

## Claims

1. An elongated medical device rotation assembly comprising:
a rotation member configured to rotate by driving an actuator, and
a support member configured to be coupled to the rotation member and support at least a portion of the elongated medical device and rotate the elongated medical device about a longitudinal axis in conjunction with the rotation of the rotation member,
wherein the support member includes an offset member for spacing at least a portion of the elongated medical device from an imaginary line extending along the rotational axis of the rotation member by a predetermined distance d on a surface on which the elongated medical device is placed.

2. The elongated medical device rotation assembly according to claim 1, **characterized in that**
the rotation member includes an opening for inserting the elongated medical device,
the support member includes an opening for inserting the elongated medical device, and
the predetermined distance d represents the maximum distance from an imaginary straight line connecting the opening of the rotation member and the opening of the support member to at least a portion of the elongated medical device.

3. The elongated medical device rotation assembly according to claim 1, **characterized in that**
the offset member includes a free roller that is axially supported in a direction perpendicular to a surface on which the elongated medical device is placed, and
the free roller includes a contact surface, at least a portion of which abuts at least a portion of the elongated medical device.

4. The elongated medical device rotation assembly according to claim 3, **characterized in that**
the free roller can freely rotate according to friction with the elongated medical device when the elongated medical device moves forward and backward in the longitudinal direction.

5. The elongated medical device rotation assembly according to claim 1, **characterized in that**
a groove is formed on the contact surface of the offset member that is in contact with at least a portion of the elongated medical device.

6. The elongated medical device rotation assembly according to claim 1, **characterized in that**
the offset member includes at least two offset members.

7. The elongated medical device rotation assembly according to claim 1, **characterized in that**
the support member further includes a movement guide, and
the offset member is installed to be movable along the movement guide.

8. A medical robot using the elongated medical device rotation assembly, comprising
a frame,
a track configured to be coupled to the frame,
a cassette platform configured to be coupled to be movable along the track,
a forward/backward assembly provided on the cassette platform and configured for forward/backward moving the elongated medical device, and
an elongated medical device rotation assembly for rotating the elongated medical device,
wherein the elongated medical device rotation assembly includes
a rotation member configured to rotate by the driving of an actuator, and
a support member configured to support the elongated medical device and rotate the elongated medical device in conjunction with the rotation of the rotation member,
wherein the support member includes an offset member for offsetting at least a portion of the elongated medical device from the rotational axis of the elongated medical device by an offset d on a surface on which the elongated medical device is placed.

9. The medical robot according to claim 8, **characterized in that**
the rotation member includes an opening for inserting the elongated medical device,
the support member includes an opening for inserting the elongated medical device, and
the offset d represents the distance from a straight line connecting the opening of the rotation member and the opening of the support member to at least a portion of the elongated medical device.

10. An elongated medical device rotation assembly comprising:
a rotation member configured to rotate by driving an actuator, and
a support member configured to support the elongated medical device and rotate the elongated medical device about a longitudinal axis in conjunction with the rotation of the rotation member,
wherein the support member includes a free roller that is axially supported in a direction perpendicular to a surface on which the elongated medical device is placed.

11. The elongated medical device rotation assembly according to claim 10, **characterized in that**
the free roller includes a contact surface, at least a portion of which abuts at least a portion of the elongated medical device.

12. The elongated medical device rotation assembly according to claim 10, **characterized in that**
when the elongated medical device is placed on the support member, at least a portion of the elongated medical device is spaced apart from the central axis in the forward and backward direction of the elongated medical device by the free roller by a maximum distance d

13. The elongated medical device rotation assembly according to claim 10, **characterized in that**
the free roller can freely rotate according to frictional force with the elongated medical device when the elongated medical device moves forward and backward in the longitudinal direction.

14. The elongated medical device rotation assembly according to claim 10, **characterized in that**
a groove is formed on the contact surface of the free roller.

15. The elongated medical device rotation assembly according to claim 10, **characterized in that**
the free roller may include at least two free rollers.

16. An elongated medical device movement assembly comprising:
a forward/backward assembly for moving the elongated medical device forward and backward and the elongated medical device rotation assembly according to claims 10 to 15.

17. A medical robot comprising the elongated medical device movement assembly according to claim 16.
